# EUROPEAN PATENT APPLICATION

(11) **EP 4 671 235 A1**
(43) Date of publication of application: **31.12.2025**
(21) Application number: 24184739.1
(22) Date of filing: 26.06.2024
(51) Int. Cl.: C07D 207/27, C07C 237/04, C07K 5/083, A61K 38/06

(54) **SYNTHESIS OF SMALL MOLECULE AGONISTS OF NEUROTROPHIN**

(71) Applicant: Oculis Operations Sàrl, 1003 Lausanne (CH)
(72) Inventor: Egilsson, Jón Freyr, 221 HAFNARFJORDUR (IS); Asgrimsdottir, Gudrun Marta, 108 REYKJAVIK (IS)
(74) Representative: Plasseraud IP

(57) **Abstract**

The disclosure concerns a process for synthesizing a compound of formula Fl: 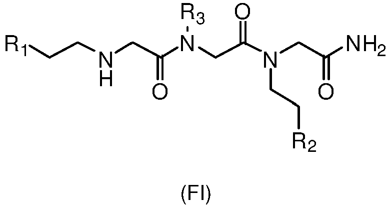 wherein:
R₁ is phenyl substituted with halogen or trifluoromethyl, and further optionally substituted with one or two substituents selected from the group consisting of halogen, (C₁-C₆)alkyl, (C₁-C₆)alkoxy, and halo(C₁-C₆)alkyl; or alternatively R₁ is pyrrolidin-1-yl;
R₂ is 2-oxo-pyrrolidin-1-ylmethyl or sulfamoylphenyl; and
R₃ is chosen from propyl, 1-methylethyl, butyl, 2-methylpropyl, pentyl, 1 -methyl-butyl, 2- methylbutyl, hexyl, 4-methylpentyl, 3-methylpentyl, 2-methylpentyl, and 1- methylpentyl. Intermediates useful for the preparation of the compounds are also provided.

## Description

### Technical Field

This disclosure pertains to the field of therapeutics for neurological, psychiatric disorders, and ageing. In particular, this disclosure relates to a synthesis route of small molecule agonists of neurotrophin (Nerve Growth Factor (NGF) or Brain-Derived Neurotrophic Factor (BDNF)), more specifically small molecules of formula FI:

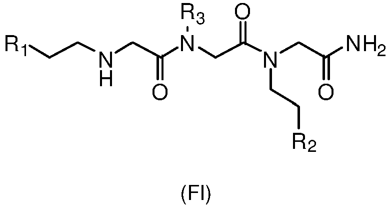

### Background Art

A synthesis route for compounds of formula FI is known. For instance, Masip et al. (Med. Chem. 13 (2005)1929 (doi:/j.bmc.2005.01.024) discloses a solid phase synthesis using positional scanning format and submonomer strategy. This synthesis route comprises 7 steps before a final step of cleavage to recover the targeted molecule from the solid support (Rink amide resin), as shown below:

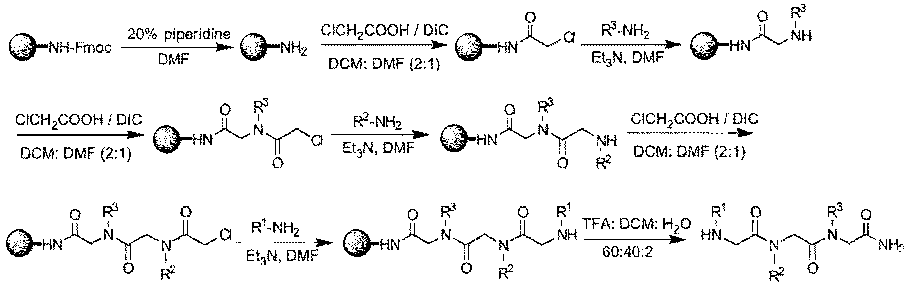

The first step of this synthesis route is the deprotection of the amine function of the solid support. The six following steps are a repetition of a sequence of two steps, namely an acylation immediately followed by an amination. Therefore, in this synthesis route, molecules are grown sequentially by using appropriate primary amines regarding the targeted molecule.

Although this synthesis step may be satisfactory in certain conditions, it would be beneficial to propose a synthesis route without using a solid support, in particular which would be more suitable for large-scale synthesis for industrial manufacturing.

### Summary

It is proposed a process for synthesizing a compound of formula FI: 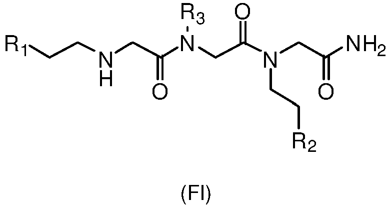 wherein:
R₁ is phenyl substituted with halogen or trifluoromethyl, and further optionally substituted with one or
two substituents selected from the group consisting of halogen, (C₁-C₆)alkyl, (C₁-C₆)alkoxy, and
halo(C₁-C₆)alkyl; or alternatively R₁ is pyrrolidin-1-yl;
R₂ is 2-oxo-pyrrolidin-1-ylmethyl or sulfamoylphenyl; and
R₃ is chosen from propyl, 1-methylethyl, butyl, 2-methylpropyl, pentyl, 1 -methyl-butyl, 2- methylbutyl, hexyl, 4-methylpentyl, 3-methylpentyl, 2-methylpentyl, and 1- methylpentyl;
comprising the steps of:
   (S1) synthesizing a compound of formula F2: 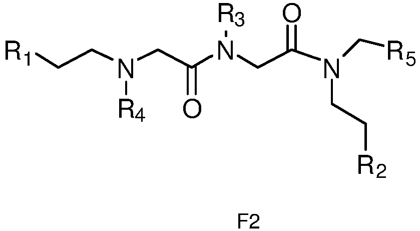
      wherein R₄ is hydrogen atom, or a protecting group selected in the group comprising, Boc, Fmoc or formyl,
      wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
(S2) if R₄ is a protecting group selected in the group comprising, Boc, Fmoc or formyl, removing said protecting group,
(S3) Amidifying R₅.

### Brief Description of Drawings

### Fig. 1 to Fig. 29

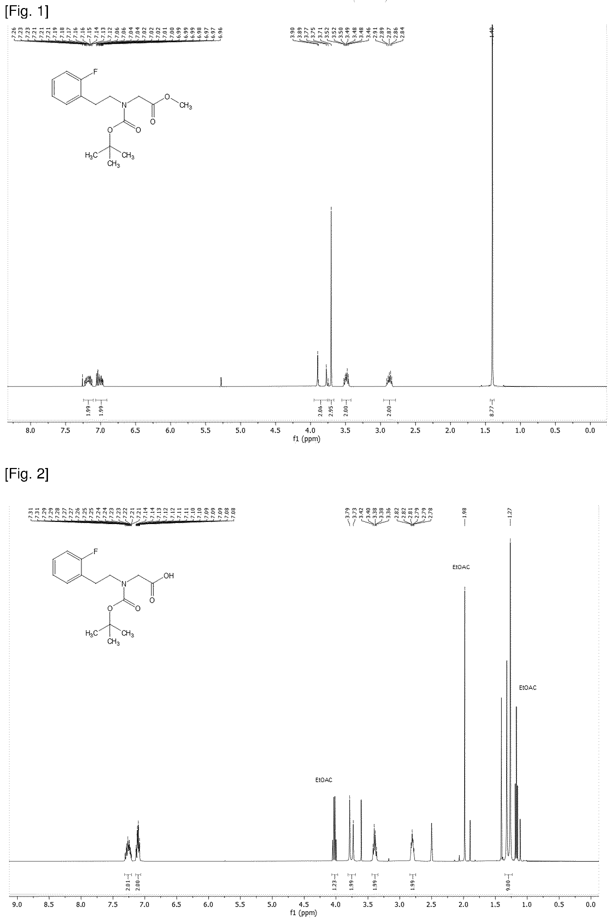

Figures 1 to figure 29 are NMR spectra of molecules synthesized in examples 1 to 29.

### Detailed description of the disclosure

As mentioned above, the disclosure concerns a process for synthesizing a compound of formula FI: 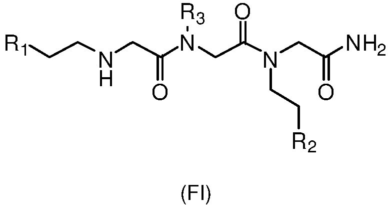 wherein:
R₁ is phenyl substituted with halogen or trifluoromethyl, and further optionally substituted with one or
two substituents selected from the group consisting of halogen, (C₁-C₆)alkyl, (C₁-C₆)alkoxy, and
halo(C₁-C₆)alkyl; or alternatively R₁ is pyrrolidin-1-yl;
R₂ is 2-oxo-pyrrolidin-1-ylmethyl or sulfamoylphenyl; and
R₃ is chosen from propyl, 1-methylethyl, butyl, 2-methylpropyl, pentyl, 1 -methyl-butyl, 2- methylbutyl, hexyl, 4-methylpentyl, 3-methylpentyl, 2-methylpentyl, and 1- methylpentyl;
comprising the steps of:
   (S1) synthesizing a compound of formula F2: 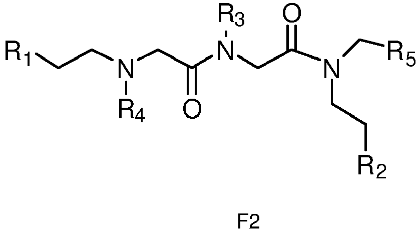
      wherein R₄ is hydrogen atom, or a protecting group selected in the group comprising, Boc, Fmoc or formyl,
      wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
(S2) if R₄ is a protecting group selected in the group comprising, Boc, Fmoc or formyl, removing said protecting group,
(S3) Amidifying R₅. *The step S1*

Step S1 may be implemented in several ways.

In an embodiment, wherein step (S1) comprises the following successive sub steps:
(S1-1a) reacting a compound of formula F3 with a compound of formula F4: 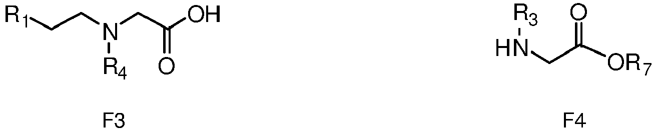
wherein R₇ being -CH₃, -CH₂CH₃; ⁱPr; -^{t}Bu, -H, or -Benzyl,
resulting in a compound of formula F5 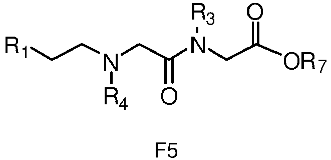
(S1-1b) reacting F5 with a compound or formula F6

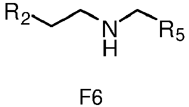

In an embodiment, step S1-1a is carried out in N-methyl-2-pyrrolidinone (NMP) at room temperature, for instance 25°C, in presence of 1.5 molar equivalent of 1,1'-carbonyldiimidazole (CDI) and 4 molar equivalent of 1 ,8-diazabicyclo[5.4.0]undec-7-ene (DBU).

In an embodiment, step S1-1b is carried out in dimethylsulfoxide (DMSO) at room temperature, for instance 25°C, in presence of 1.5 molar equivalent of 1,1'-carbonyldiimidazole (CDI) and 4 molar equivalent of 1 ,8-diazabicyclo[5.4.0]undec-7-ene (DBU).

Alternatively, step (S1-1a) comprises the following successive sub steps:
(S1-1aa) reacting a compound of formula F3 with a compound of formula F7: 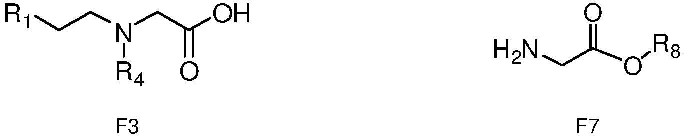
wherein R₈ being -CH₃, -CH₂CH₃; ⁱPr; -^{t}Bu, -H, or -Benzyl,
resulting in a compound of formula F8 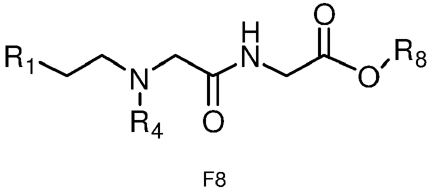
(S1-1ab) reacting F8 with a compound or formula F9 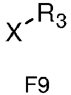
wherein X is a halogen atom,
resulting in a compound of formula F10 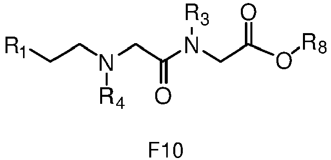
(S1-1ac) if R₈ is different from H, and R₇ is H, hydrolyzing F10 resulting in a compound of formula F5.

In an embodiment, step S1-1aa is carried out in dichloromethane (DCM) at a temperature around between 30°C and 50°C, in presence of 1.5 molar equivalent of benzotriazole-1-yl-oxy-tris-(dimethylamino)-phosphonium hexafluorophosphate (BOP) and 2 molar equivalent of triethylamine.

In an embodiment, step S1-1ab is carried out in dichloromethane (DCM) at a temperature around between 30°C and 50°C, in presence of 1 molar equivalent of benzotriazole-1-yl-oxy-tris-(dimethylamino)-phosphonium hexafluorophosphate (BOP) and 1 molar equivalent of triethylamine.

In an embodiment, compound of formula F6 may be synthesized by reacting a compound of formula F11 with a compound of formula F12 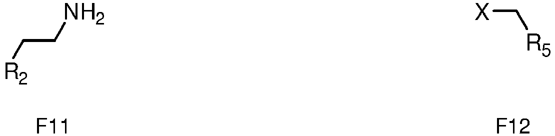 wherein X is a halogen atom.

For instance, if R₅ is CN, compound of formula F6 may be synthesized by reacting compound of formula F11 with compound of formula F12 in triethylamine and acetonitrile (ACN) at room temperature, for instance around 25°C.

For instance, if R₅ is -C(O)OBn, compound of formula F6 may be synthesized by reacting compound of formula F11 with compound of formula F12 in N,N-diisopropylethylamine (DIPEA) and acetonitrile (ACN) at room temperature, for instance around 25°C.

Alternatively, compound of formula F3 is synthesized by reacting a compound of formula F13 with a compound of formula F14 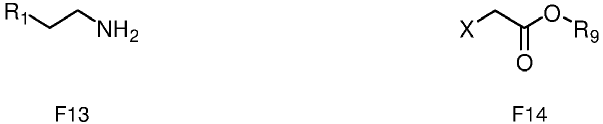
wherein X is a halogen atom, and
wherein R₉ being -CH₃, -CH₂CH₃; ⁱPr; -^{t}Bu, -H, or -Benzyl,
resulting in a compound of formula F15, 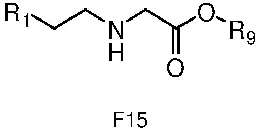
protecting the amine function of said compound of formula F15 with a protecting group selected in the group comprising, Boc, Fmoc and formyl, resulting in a compound of formula F16 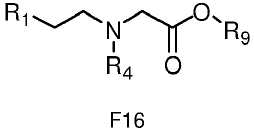 hydrolyzing F16.

For instance, if R₉ is Me, compound of formula F15 may be synthesized by reacting compound of formula F13 with compound of formula F14 in triethylamine and acetonitrile (ACN) at room temperature, for instance around 25°C.

Then, the amine function of compound of formula F15 may be protected, for instance with a Boc, by reacting compound of formula F15 with 1.05 molar equivalent of di-tert-butyl-dicarbonate, with 1.5 molar equivalent of triethylamine in dichloromethane (DCM) at room temperature, for instance around 25°C, resulting in compound od formula F16.

Compound of formula F16 may be hydrolyzed in methanol at room temperature, for instance around 25°C, in the presence of 3.0 molar equivalent of sodium hydroxide.

In another embodiment, step (S1) comprises the following successive sub steps: (S1-2a) reacting a compound of formula F17 with a compound of formula F3:

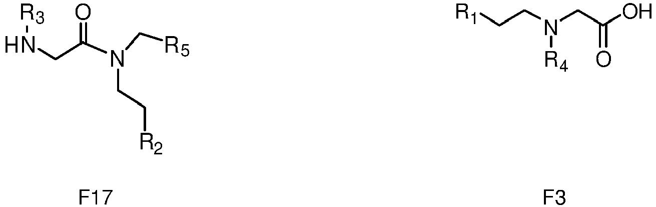

In an embodiment, step S1-2a is carried out in dichloromethane (DCM) at a temperature around between 30°C and 50°C, in presence of between 1 and 2, preferably around 1.5 molar equivalent of benzotriazole-1-yl-oxy-tris-(dimethylamino)-phosphonium hexafluorophosphate (BOP) and between 2 and 4 molar equivalent of triethylamine.

In another embodiment, step (S1) comprises the following successive sub steps:
(S1-3a) reacting a compound of formula F17 with a compound of formula F18: 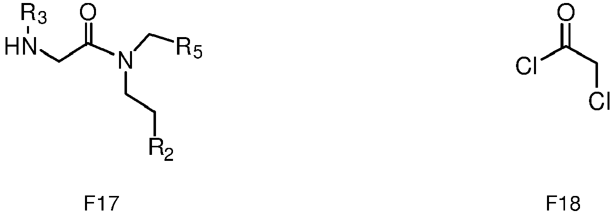
resulting in a compound of formula F19 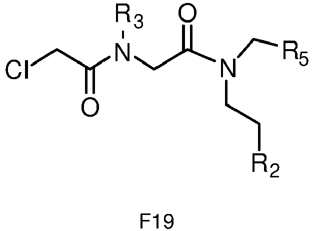
reacting F19 with a compound of formula F13:

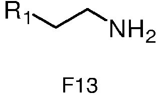

In an embodiment, step S1-3a is carried out in dichloromethane (DCM) at a temperature around between 20°C and 50°C, in presence of triethylamine to obtain compound of formula F19. Then, compound of formula F19 reacts with compound of formula F13, in acetonitrile at a temperature around between 20°C and 50°C, in presence of triethylamine.

Alternatively, compound of formula F17 may be synthesized by reacting a compound of formula F6 with a compound of formula F20 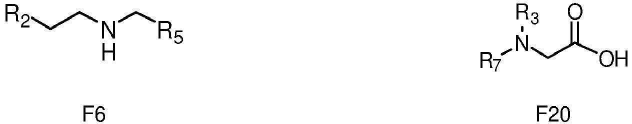 wherein R₇ is a protecting group selected from BOC, FMOC, formyl or benzyl, and removing said protecting group.

For instance, if R₇ is BOC, compound of formula F17 may be synthesized by reacting compound of formula F6 with compound of formula F20 in dichloromethane (DCM) at a temperature around between 25°C and 50°C, in presence of between 1 and 2, preferably 1.5, molar equivalent of benzotriazole-1-yl-oxy-tris-(dimethylamino)-phosphonium hexafluorophosphate (BOP) and between 2 and 3 molar equivalent of triethylamine. Then BOC may be removed, for instance in isopropanol at a temperature around between 20°C and 50°C in the presence of HCl.

Alternatively, compound of formula F17 may be synthesized by reacting a compound of formula F6 with a compound of formula F18 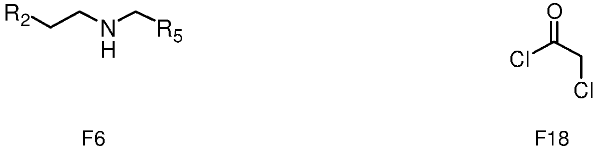
resulting in a compound of formula F21 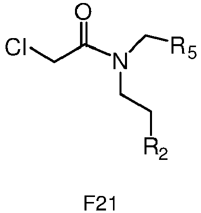
reacting F21 with a compound of formula F22

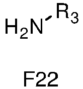

Compound of formula F6 may react with compound of formula F18 in dichloromethane (DCM) at room temperature, for instance around 25°C, in presence of triethylamine to obtain compound of formula F21. Then, compound of formula F21 reacts with compound of formula F22, in acetonitrile at room temperature, for instance around 25°C in presence of triethylamine.

### Step (S2)

According to the process of the present disclosure, if R₄ is a protecting group selected in the group comprising, Boc, Fmoc or formyl, a step S2 of removing said protecting group should be caried out. Step S2 may be carried out by any conventional way known to those skilled in the art.

For instance, if R₄ is BOC, it may be removed, for instance in isopropanol at a temperature around between 20°C and 60°C in the presence of HCl, or in methanol at room temperature, for instance around 25°C in the presence of sulfuric acid.

For instance, if R₄ is formyl, it may be removed, for example in methanol at a temperature around between 20°C and 50°C in the presence of sulfuric acid.

### Step (S3)

According to the process of the present disclosure, step S3 consists of amidifying R₅.

In an embodiment, step S3 may be carried out in methanol at a temperature around between 20°C and 60°C in the presence of ammonia.

### The compound of formula FI

According to the present disclosure, compound of formula FI is the following: 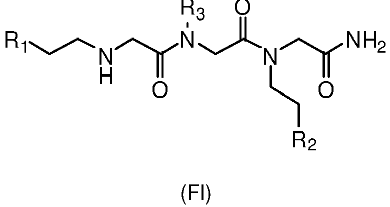 wherein:
R₁ is phenyl substituted with halogen or trifluoromethyl, and further optionally substituted with one or
two substituents selected from the group consisting of halogen, (C₁-C₆)alkyl, (C₁-C₆)alkoxy, and
halo(C₁-C₆)alkyl; or alternatively R₁ is pyrrolidin-1-yl;
R₂ is 2-oxo-pyrrolidin-1-ylmethyl or sulfamoylphenyl; and
R₃ is chosen from propyl, 1-methylethyl, butyl, 2-methylpropyl, pentyl, 1 -methyl-butyl, 2- methylbutyl, hexyl, 4-methylpentyl, 3-methylpentyl, 2-methylpentyl, and 1- methylpentyl.

When R₁ is (C₁-C₆)alkyl, it may be methyl, ethyl, propyl, butyl, 2-methylpropyl, pentyl, 2 methylbutyl, 2,2-dimethylpropyl, hexyl, 2-methylpentyl, 3-methylpentyl, 2,2-dimethylbutyl, 2,3-dimethylbutyl, R₁ being linked by any carbon.

When R₁ is (C₁-C₆)alkoxy, it may be -O-R₁₂, wherein R₁₂ may be methyl, ethyl, propyl, butyl, 2-methylpropyl, pentyl, 2 methylbutyl, 2,2-dimethylpropyl, hexyl, 2-methylpentyl, 3-methylpentyl, 2,2-dimethylbutyl, 2,3-dimethylbutyl, R₁₂ being linked by any carbon to the oxygen, and R₁ being linked by the oxygen.

When R₁ is halo(C₁-C₆)alkyl, it may be -X-R₁₂, wherein X is a halogen atom, preferably selected from fluorine, chlorine and bromine, wherein R₁₂ may be methyl, ethyl, propyl, butyl, 2-methylpropyl, pentyl, 2 methylbutyl, 2,2-dimethylpropyl, hexyl, 2-methylpentyl, 3-methylpentyl, 2,2-dimethylbutyl, 2,3-dimethylbutyl, R₁₂ being linked by any carbon to the halogen atom, and R₁ being linked by the halogen.

In a preferred embodiment, R₁ is 2-fluorophenyl, R₂ is 2-oxo-pyrrolidin-1-ylmethyl and R₃ is 2-methylpropyl. Thus, in a preferred embodiment, compound of formula FI is the following:

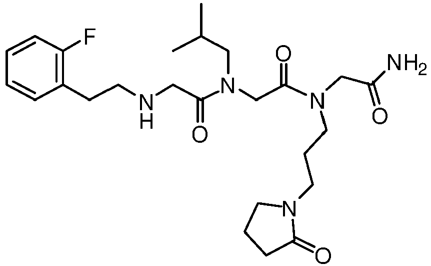

### Intermediate compounds

The process according to the disclosure involves several intermediate compounds. Among them, some are of particular interest.

As such, the disclosure also pertains to the following compounds of formula F23, F24, F25, F26, F27, F28, F29 and F30: 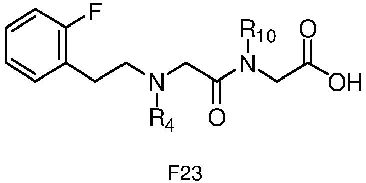
wherein R₁₀ is -H or isobutyl,
and salts thereof, 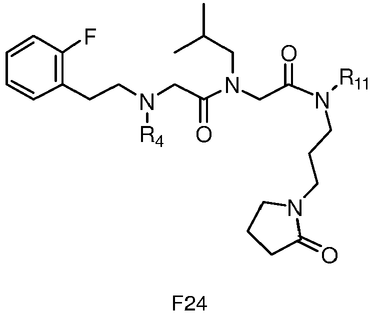
wherein R₁₁ is -H or -CH₂R₅,
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof, 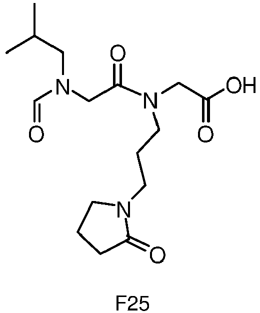
and salts thereof, 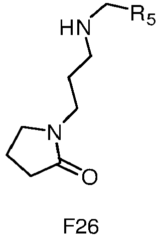
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof, 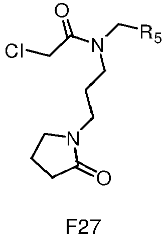
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof, 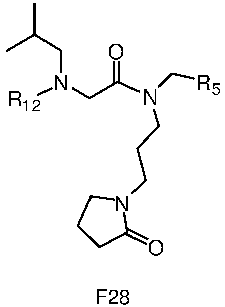
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl, and
wherein R12 is hydrogen atom, BOC, FMOC, formyl, Bn or COCH₂Cl,
and salts thereof, 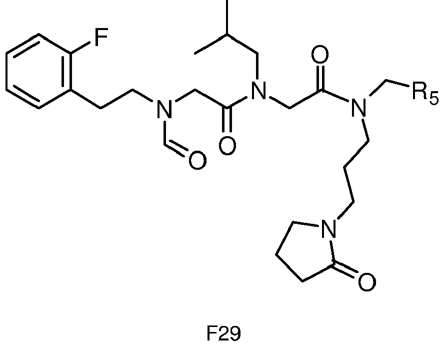
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof, and 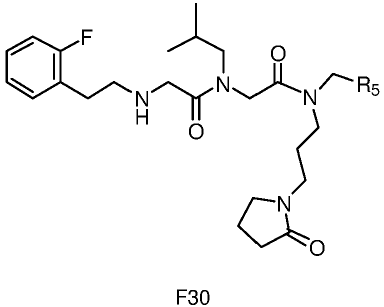
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof.

### Pharmaceutical compositions

The disclosure also concerns pharmaceutical compositions comprising the compound of formula FI obtained by the process described above, and optionally one or more pharmaceutically acceptable excipient(s).

In an embodiment, the pharmaceutical composition comprises less than 0.3% of each individual impurity.

### Examples

### Materials and methods:

LC/MS analyses were recorded on a Waters UPLC Acquity system using the following parameters:
Column: Acquity BEH C18 1.7 µm 2.1*150mm;
3 min run
Photodiode array detection from 210 to 400 nm, sum of absorbance at all wavelengths

MS Detection: Waters Acquity Qda (single quadrupole) detector with electrospray ionization (ESI) in positive and negative mode with scan range m/z 85-850

### Solvent system:

- Mobile phase A: Water/MeCN 95/5 + 0.1% Formic Acid
- Mobile phase B: MeCN + 0.1% Formic Acid

### Gradient:

| **Time (min)** | **Flow (ml/min)** | **%A** | **%B** |
|---|---|---|---|
| **0** | 0.5 | 98 | 2 |
| **0.10** | 0.7 | 98 | 2 |
| **1.40** | 0.7 | 5 | 95 |
| **2.20** | 0.7 | 5 | 95 |
| **2.30** | 0.7 | 98 | 2 |
| **2.50** | 0.5 | 98 | 2 |
| **3.00** | 0.5 | 98 | 2 |

¹H NMR and ¹³C NMR were recorded on a Bruker Avance 400 MHz, 100 MHz. Chemical shifts are expressed in parts per million (ppm) downfield from residual solvent peaks, and coupling constants are reported in hertz (Hz).

Multiplicities of signals in ¹H NMR spectra are reported using the following abbreviations: br: broad, s = singlet, d = doublet, t = triplet, q = quartet, hep = heptet, dt = doublet of triplets, dq = doublet of quartets, td = triplet of doublets, tt = triplet of triplets, m = multiplet.

The following compounds, set forth in table below have been synthesized as reflected in examples 1 to 29 below:

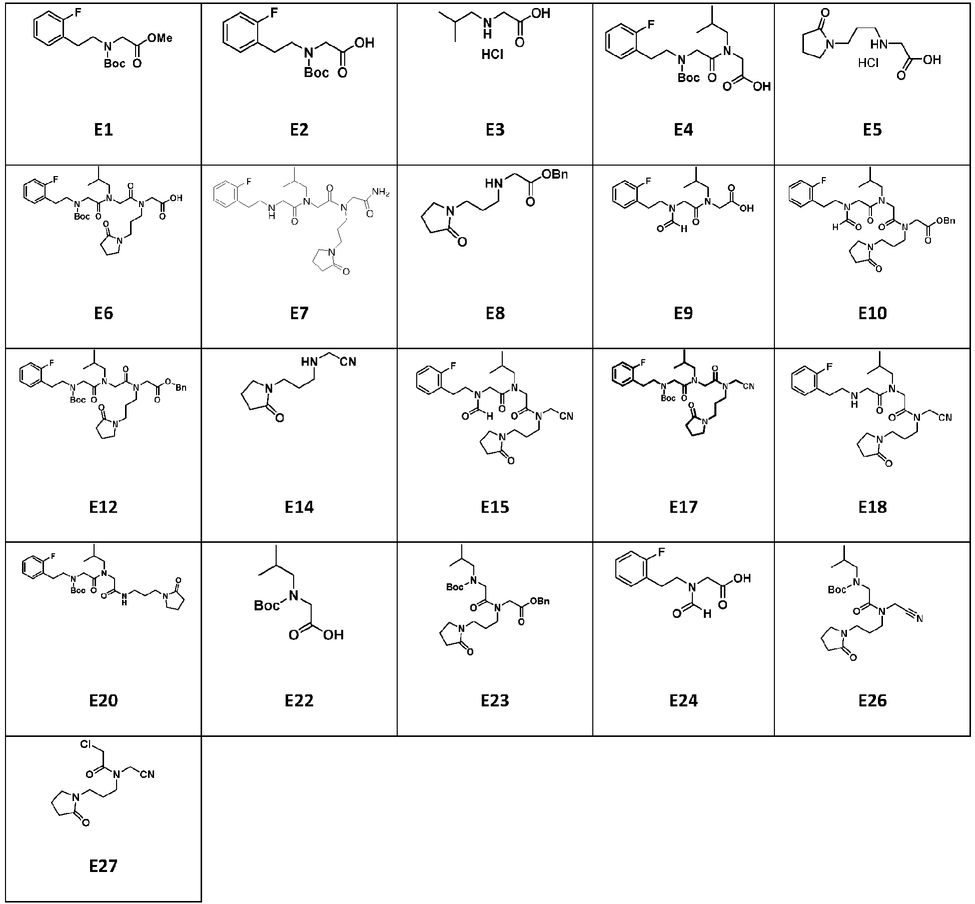

### Example 1: Synthesis of a compound of formula F16:

In a 100 mL round bottom glass flask, 2-(2-fluorophenyl)ethan-1-amine (7.00 g, 0.050 mol, 1.00 eq) was solubilized in acetronitrile(100 mL). Triethylamine was then added (15.4 mL, 0.111 mol, 2.20 eq), followed by methyl-2-bromoacetate (7.1 mL, 0.075 mol, 1.50 eq). The reaction mixture was stirred for 2 hours at 25°C.

LCMS analysis after this time showed complete conversion. The reaction was quenched with addition of a saturated aqueous NH₄Cl solution and extracted with DCM. The organic layer was washed twice with NH₄Cl solution and concentrated till dryness to afford crude methyl (2-fluorophenethyl)glycinate (10.20 g, 0.036 mol, 72% yield) which was used as such for the next step.

In a 500 mL round bottom flask, methyl (2-fluorophenethyl)glycinate (10.20 g, 0.036 mol, 1.000 eq) was solubilized in DCM (123 mL) and triethylamine was added (7.6 mL, 0.054 mol, 1.50 eq). Then, di-tert-butyl-dicarbonate was added portionwise, and reaction mixture was stirred for 16 hours at 25°C (orange solution).

LCMS analysis after this time showed complete conversion. The reaction mixture was hydrolyzed with water and phases were separated. The organic layer was washed twice with water then with 2M HCl three times. The organic layer was dried over Na₂SO₄, filtered, and evaporated to dryness to afford E1 as an orange oil (11.14 g, 0.035 mol, 69% yield over 2 steps).

LC purity: 98.5 A%, MS (positive ESI, [M+H]+): 312.1, ¹H NMR (400 MHz, CDCl3) (figure 1) δ 7.19 (ddd, J = 12.4, 6.9, 3.8 Hz, 2H), 7.08 - 6.93 (m, 2H), 3.84 (d, J = 49.6 Hz, 2H), 3.71 (s, 3H), 3.49 (dt, J = 10.6, 7.4 Hz, 2H), 2.87 (dt, J = 14.1, 7.5 Hz, 2H), 1.40 (s, 9H).

### Example 2: Synthesis of a compound of formula F3:

In a 50 mL round bottom flask, methyl N-(tert-butoxycarbonyl)-N-(2-fluorophenethyl)glycinate E1 (9.80 g, 0.030 mol, 1.000 eq) was dissolved in MeOH (85 mL). Then, an aqueous solution of NaOH 30% (6.0 mL, 0.060 mol, 2.000 eq) was added and reaction mixture was stirred 2 hours at 25°C. LCMS analysis after this time showed complete conversion. The reaction mixture was quenched with water, and EtOAc was added. The organic layer was washed with aqueous saturated NH₄Cl solution twice, and then with 1M HCl. The organic phase was dried over Na₂SO₄, and concentrated till dryness to afford E2 as an orange oil (9.80 g, 0.028 mol, 79% yield) with 15% w/w of residual EtOAc.

LC purity: 99.0 A%, MS (negative ESI, [M-H]-): 296.1, ¹H NMR (400 MHz, DMSO) (Figure 2) δ 7.37 - 7.18 (m, 2H), 7.11 (dtd, *J* = 8.6, 6.9, 1.6 Hz, 2H), 3.76 (d, *J* = 22.1 Hz, 2H), 3.39 (q, *J* = 7.4 Hz, 2H), 2.80 (td, *J* = 7.0, 4.0 Hz, 2H), 1.27 (s, 9H).

### Example 3: Synthesis of a compound of formula F4:

In a 250 mL round bottom three-neck glass reactor was loaded isobutylamine (10.2 mL, 0.103 mol, 1.000 eq) followed by water (30 mL). At 25°C, formic acid (5.4 mL, 0.144 mol, 1.400 eq) was slowly added (exothermic addition) keeping the internal temperature below 50°C.

This reaction mixture was stirred while going back to 35°C, and then glyoxylic acid monohydrate (28.3 g, 0.307 mol, 3.000 eq) solubilized in water (30 mL) was slowly added. The reaction temperature reached 45°C, turned yellow and a small degassing was observed. This solution was then heated at 50°C for 1 hour. After this time hydrochloric acid (37% aqueous solution, 13 mL, 0.151 mol, 1.470 eq) was added and the solution was stirred at 80°C for 16 hours.

The reaction mixture was then partially concentrated to a low volume in a rotary evaporator, and residual water was swapped several times with MeCN until an off-white solid was obtained. The solid was triturated with MeCN at 20°C for 30 minutes, filtered and dried in an oven at 45°C under vacuum to afford E3 (13.0 g, 0.778 mol, 76% yield).

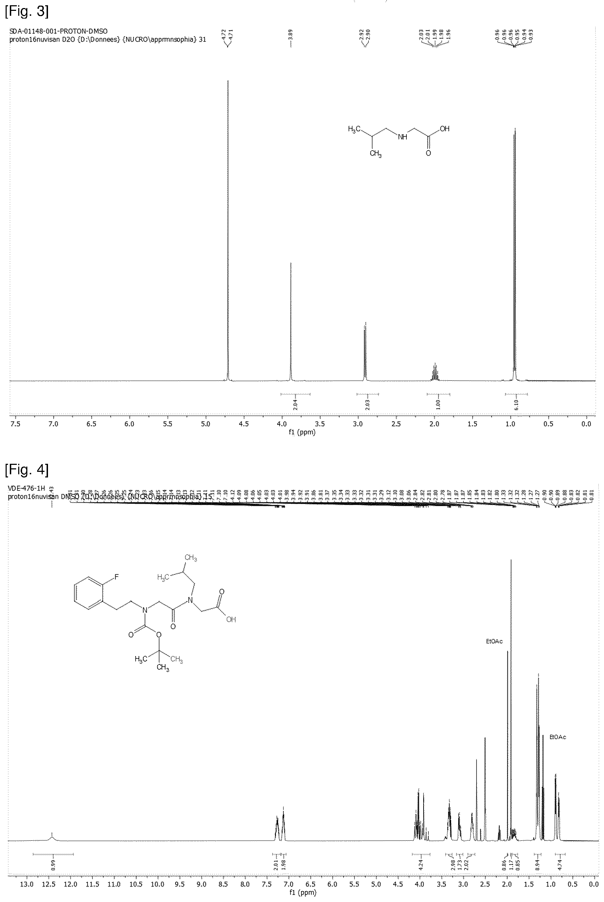

MS (positive ESI, [M+H]+): 132.2, ¹H NMR (400 MHz, D2O) (Figure 3) δ 3.97 (s, 2H), 2.99 (d, J = 7.3 Hz, 2H), 2.07 (dp, J = 13.8, 6.9 Hz, 1H), 1.03 (d, J = 6.7 Hz, 6H).

### Example 4: Synthesis of a compound of formula F5:

In a 50 mL round bottom flask, 3 (4.05 g, 0.013 mol, 1.00 eq) was dissolved in NMP (19 mL). At 25°C, CDI (3.08 g, 0.019 mol, 1.50 eq) was added portionwise within 5 minutes, and the reaction was stirred 30 minutes. Then, 4 (4.25 g, 0.025 mol, 2.00 eq) was added, followed by DBU (7.6 mL, 0.051 mol, 4.00 eq). The reaction mixture was stirred at 25°C for 2 hours. The reaction mixture was quenched with EtOAc, and 2M HCl. Phases were separated and the aqueous layer was extracted twice with EtOAc. The combined organic layers were washed with 1M aqueous HCl, dried over Na2SO4, filtered, and evaporated till dryness to afford E4 (5.90 g, 0.013 mol, 93% yield) containing 6% w/w of residual EtOAc.

LC purity: 94.0 A%, MS (negative ESI, [M-H]-): 409.4, ¹H NMR (400 MHz, DMSO) (Figure 4) δ 12.43 (s, 1H), 7.40 - 7.20 (m, 2H), 7.13 (tq, J = 7.2, 2.8 Hz, 2H), 4.16 - 3.76 (m, 4H), 3.33 (ddd, J = 14.1, 11.2, 7.1 Hz, 2H), 3.09 (dd, J = 13.8, 7.4 Hz, 2H), t2.80 (dd, J = 9.5, 5.4 Hz, 2H), 1.88 - 1.71 (m, 1H), 1.52 - 1.22 (m, 9H), 0.94 - 0.71 (m, 6H).

### Example 5: Synthesis of a compound of formula F6:

In a 500mL flask was introduced 1-(3-aminopropyl)-pyrrolidin-2-one (15.0g, .10 mol, 1.00 eq) followed with H2O (75.0 mL). Solution was stirred for 5min at 23°C before formic acid (5.8 mL, 0.15 mol, 1.40 eq) was added dropwise in 5 min leading to an exotherm. Internal temperature reached 35°C. 10min later, at 23°C, glyoxylic acid (29.1 g, 0.32 mol, 3.00 eq) solution in water (37.5 mL) was added dropwise over 5min.

Solution was stirred at 23°C for 10min, then at 38°C for 2h and finally at 60°C for 4h30. Then a 37% HCl aqueous solution (12.8 mL, 0.15 mol, 1.50 eq) was added dropwise and the reaction mixture was stirred at 82°C for 16h. The Solution was cooled to 40°C and evaporated to dryness under reduced pressure. The residue was then co-evaporated several time with toluene to remove all traces of water. The residue was then dissolved in 30 mL of hot iPrOH and then allowed to cool to 23°C over 16 hours while the product crystallized. The solid was collected by filtration and dried under vacuum at 45°C for 16 hours before being triturated in MeCN (100.0 mL) at 50°C for 2h. The solid was collected by filtration rinsed once with ACN and dried under vacuum at 45°C overnight to afford E5 (18.2 g, 0.07 mol, 69% yield) as a white solid.

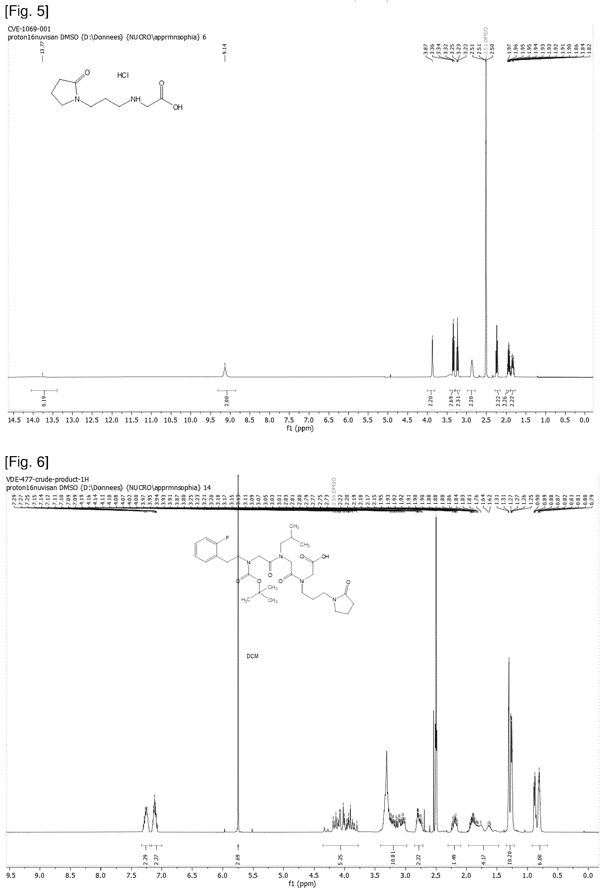

MS (negative ESI, [M-H]-): 199.1, ¹H NMR (400 MHz, DMSO) (Figure 5) δ 13.77 (s, 0H), 9.14 (s, 2H), 3.87 (s, 2H), 3.34 (t, J = 7.0 Hz, 2H), 3.23 (t, J = 6.7 Hz, 2H), 2.88 (d, J = 8.2 Hz, 2H), 2.33 - 2.15 (m, 2H), 1.94 (qd, J = 8.1, 6.6 Hz, 2H), 1.84 (p, J = 6.9 Hz, 2H).

### Example 6: Synthesis of a compound of formula F2:

In a 100 mL round bottom flask, was E4 (1.00 g, 2.43 mmol, 1.00 eq) and DMSO (4 mL). To this solution was added CDI (594 mg, 3.65 mmol, 1.50 eq) portionwise and reaction mixture was stirred half an hour at 22°C. Then, E5 (976 mg, 4.87 mmol, 2.00 eq) was added and stirring was maintained for 10 minutes. DBU (1.5 mL, 9.72 mmol, 4.00 eq) was finally added with small exotherm noticed, up to 26°C. The reaction mixture was heated near 40°C, and conditions were maintained for 22 hours. LCMS analysis after this time showed complete conversion. Solution was cooled down near 10°C and hydrolyzed with 6M aqueous HCl solution. The reaction mixture was diluted with water and extracted with DCM. The organic layer was then washed with a 0.5 M HCl solution and finally with water and brine. The organic layer was dried over Na₂SO₄, filtered and concentrated till dryness to afford E6 (1.26 g, 2.12 mmol, 65% yield) as an oil with 14% w/w of residual DCM.

LC purity: 93.0 A%, MS (negative ESI, [M-H]-): 591.3, ¹H NMR (400 MHz, DMSO) (Figure 6) δ 7.41 - 7.19 (m, 2H), 7.18 - 7.02 (m, 2H), 4.21 - 3.73 (m, 6H), 3.26 - 2.92 (m, 10H), 2.88 - 2.73 (m, 2H), 2.30 - 1.55 (m, 7H), 1.59 - 1.10 (m, 9H), 1.03 - 0.57 (m, 6H).

### Example 7: Synthesis of a compound of formula F1:

In a 25 mL round bottom flask, was charged E6 (240 mg, 0.324 mmol, 1.00 eq) followed by iPrOH (3.0 mL). Then a 5.5M HCl solution in iPrOH (236 µL, 1.296 mmol, 4.00 eq) was added and the reaction mixture was stirred at 40°C for 16 hours. LCMS analysis after this time showed complete Boc-cleavage and a mixture of isopropylester and carboxylic acid intermediates. The reaction mixture was concentrated till dryness under reduced pressure to afford an oily residue. This residue was dissolved in MeOH (1.0 mL) and a 7M NH₃ solution in MeOH was added (2.0mL). The solution was stirred at 50°C for 24h. The solution was evaporated to dryness under reduced pressure and the residue was purified by chromatography over SiO₂ eluting with DCM/EtOH 98:2 to 90:10. Purest fractions were combined and evaporated to dryness to afford compound of formula F1 (E7) (38mg, 19 % yield) as a white solid.

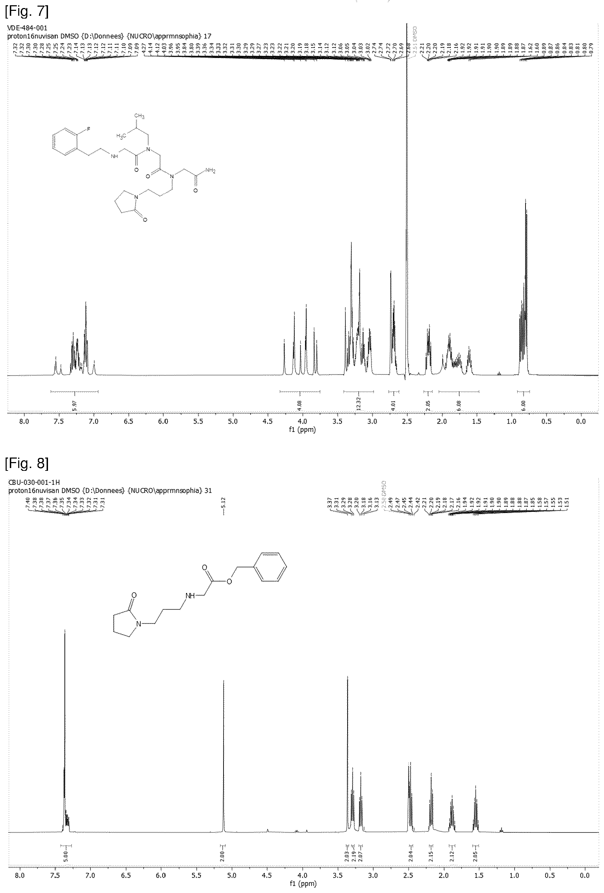

LC purity: 97.0 A%, MS (positive ESI, [M+H]+): 492.4, ¹H NMR (400 MHz, DMSO, mixture of rotamers) (Figure 7) δ 7.70 - 6.88 (m, 7H), 4.37 - 3.68 (m, 4H), 3.46 - 2.99 (m, 10H), 2.79 - 2.63 (m, 4H), 2.41 - 2.11 (m, 2H), 2.05 - 1.43 (m, 5H), 1.10 - 0.55 (m, 6H).

### Example 8: Synthesis of a compound of formula F6:

In a 250mL flask was introduced 1-(3-aminopropyl)pyrrolidin-2-one (7.00 g, 0.049 mol, 1.00 eq) followed by MeCN (105.0 mL). Benzyl 2-bromoacetate (12.50 g, 0.055 mol, 1.11 eq) was added to the reaction mixture (exotherm) followed by DIPEA (25.7 mL, 0.148 mol, 3.00 eq). Precipitation was observed. The reaction mixture was stirred at 23°C for 15h. LCMS analysis after this time showed complete conversion with formation of mono and bis-alkylated compound in a 65/35 ratio. The reaction was quenched by addition of DCM (30 mL) and water (30 mL). Layers were separated and the aqueous phase was extracted DCM(2x30mL). Combined organic phases were washed with a saturated aqueous NaCl solution (2x15mL), dried over Na₂SO₄ and concentrated to dryness under reduced pressure to afford 12.9g of a yellowish oil. This residue was purified by chromatography over SiO₂ eluting with DCM/EtOH 98:2 to 90:10. Purest fractions were combined and evaporated to dryness to afford E8 (6.1g, 43% yield) as a colorless oil.

LC purity: 97.3 A%, MS (positive ESI, [M+H]+): 226.2, ¹H NMR (400 MHz, DMSO) (Figure 8) δ 7.42 - 7.29 (m, 5H), 5.12 (s, 2H), 3.37 (s, 2H), 3.29 (t, J = 7.0 Hz, 2H), 3.18 (t, J = 7.1 Hz, 2H), 2.47 (t, J = 7.0 Hz, 2H), 2.18 (t, J = 8.1 Hz, 2H), 1.95 - 1.83 (m, 2H), 1.55 (p, J = 7.0 Hz, 2H).

### Example 9: Synthesis of a compound of formula F10:

In a 2000mL four neck round bottom flask was introduced E24 (5.00 g, 0.022 mol, 1.00 eq) followed by NMP (25.0 mL). To this suspension was added portionwise CDI (5.40 g, 0.033 mol, 1.50 eq) in 25min (and reaction mixture was stirred at 23°C for 1h.

Then, E3 (7.4 g, 0.044 mol, 2.00 eq) was added portionwise and 5 min later DBU (13.3 mL, 0.088 mol, 4.00 eq) (exotherm observed but controlled with an ice bath). Internal temperature reached 28°C and the reaction mixture was stirred 10min at 23°C. and then 3h at 32°C. The reaction was quenched by the addition of 6M HCl (20.3 mL, 0.121 mol, 5.50 eq) under ice bath cooling. pH 1 was reached. The solution was diluted with 40mL of EtOAc and transferred into a separating funnel. Liquid liquid extraction was performed and aqueous layer was extracted once more with 40mL of EtOAc while organic layer was washed once more with 30mL 0.5M HCl aqueous solution. Organic layers were combined, washed twice with 30mL of water and evaporated to dryness under reduced pressure. The residue was then solubilized in 20 mL of iPrOH and then 50 mL of water were slowly added at 23°C. The solution was then stirred at 23°C for 24h while the product crystallizes. The solid was collected by filtration, washed with water and dried under vacuum at 45°C for 18h to afford E9 (4.0 g, 0.012 mol, 55% yield) as a white solid.

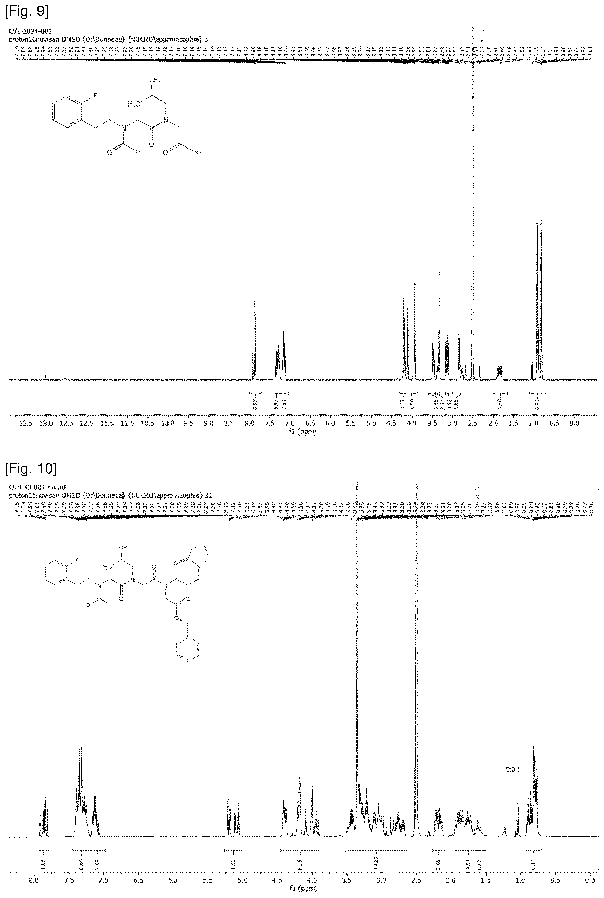

LC purity: 98.0 A%, MS (negative ESI, [M-H]-): 337.1, ¹H NMR (400 MHz, DMSO, mixture of rotamers) (Figure 9) δ 13.26 - 12.06 (br, 1H), 7.97 - 7.76 (m, 1H), 7.39 - 7.21 (m, 2H), 7.15 (qdd, J = 7.5, 4.3, 2.2 Hz, 2H), 4.33 - 4.14 (m, 2H), 4.02 (dd, J = 70.4, 4.5 Hz, 2H), 3.59 - 3.35 (m, 2H), 3.24 - 3.03 (m, 2H), 2.88 - 2.71 (m, 2H), 1.98 - 1.51 (m, 1H), 1.09 - 0.70 (m, 6H).

### Example 10: Synthesis of a compound of formula F2:

In a 50mL flask was introduced E8 (1.000 g, 0.003 mol, 1.000 eq) followed by DCM (5.0 mL). To this solution was added E9 (1.716 g, 0.006 mol, 2.000 eq) and the temperature was raised to 40°C. BOP (1.307 g, 0.003 mol, 1.000 eq) was added followed by EtsN (0.411 mL, 0.003 mol, 1.000 eq). The solution was stirred at 40°C for 2h.

LCMS analysis after this time showed complete conversion. The reaction was quenched by addition of DCM (20 mL) and water (20 mL). Layers were separated and the organic phase was washed with water (2x30 mL) and then with 1N HCl (2x20 mL). The organic phase was then dried over Na₂SO₄ and concentrated to dryness under reduced pressure to afford 1.54g of an orange oil.

This residue was purified by chromatography over SiO₂ eluting with DCM/EtOH/Et₃N (95/5/0.1). Purest fractions were combined and evaporated to dryness to afford E10 (1.2g, 48% yield) as a yellowish oil.

LC purity: 95.3 A%, MS (positive ESI, [M+H]+): 611.5, ¹H NMR (400 MHz, DMSO, mixture of rotamers) (Figure 10) δ 8.05 - 7.72 (m, 1H), 7.55 - 7.22 (m, 7H), 7.16 - 6.98 (m, 2H), 5.38 - 4.92 (m, 2H), 4.56 - 3.88 (m, 6H), 3.60 - 2.62 (m, 12H), 2.26 - 2.10 (m, 2H), 1.97 - 1.67 (m, 4H), 1.68 - 1.51 (m, 1H), 0.93 - 0.74 (m, 6H).

### Example 11: Synthesis of a compound of formula F1:

In a 25mL flask was introduced CBU-043-001 (0.500 g, 0.001 mol, 1.000 eq) followed by MeOH (3.0 mL) and conc. H₂SO₄ (0.218 mL, 0.004 mol, 5.000 eq). The solution was stirred at 50°C for 16h.

LCMS analysis after this time showed complete conversion. The reaction was quenched by addition of DCM (10 mL) and a 2.5M K₃PO₄ aqueous solution (2.6 mL). Layers were separated and the organic phase was washed with water (2x10 mL) and then with 1N HCl (2x10 mL). The organic phase was then dried over Na₂SO₄ and concentrated to dryness under reduced pressure to afford yellow oil.

This residue was dissolved in MeOH (0.5 mL) and a 7M NH₃ solution in MeOH was added (1.5 mL). The solution was stirred at 50°C for 24h.

LCMS analysis after this time showed complete conversion into compound of formula F1. The solution was evaporated to dryness under reduced pressure and the residue was diluted in EtOAc (5 mL) and warmed to 50°C. The solution was stirred at 50°C for 1h and allowed to cool to 23°C for 24h while the product crystallized. The solid was collected by filtration, washed with DIE (5 mL) and dried under vacuum at 45°C for 24h to afford compound of formula F1 (E7) (70mg, 44% yield).

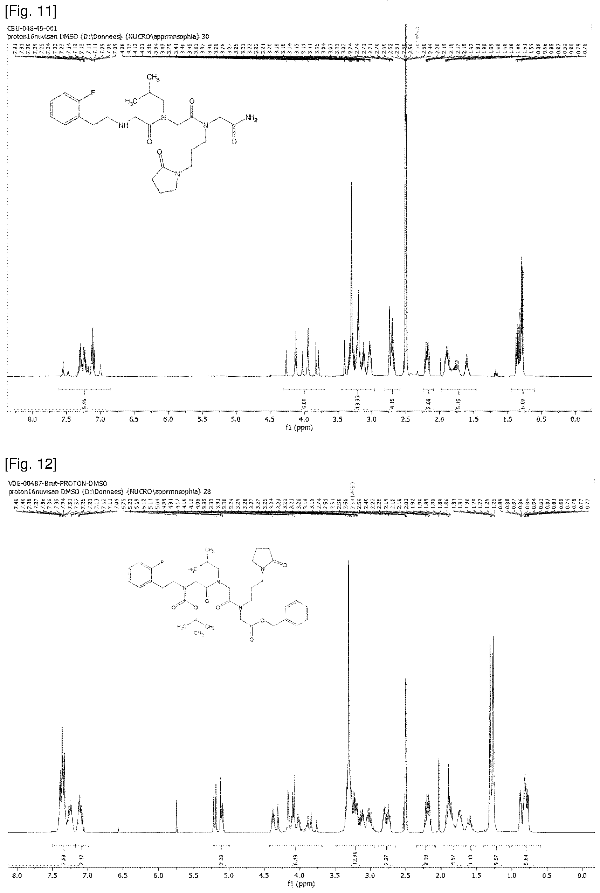

LC purity: 97.5 A%, MS (positive ESI, [M+H]+): 492.4, ¹H NMR (400 MHz, DMSO, mixture of rotamers) (Figure 11) δ 7.70 - 6.88 (m, 7H), 4.37 - 3.68 (m, 4H), 3.46 - 2.99 (m, 10H), 2.79 - 2.63 (m, 4H), 2.41 - 2.11 (m, 2H), 2.05 - 1.43 (m, 5H), 1.10 - 0.55 (m, 6H).

### Example 12: Synthesis of a compound of formula F2:

In a 25 mL sealed glass vial, E4 (595 mg, 1.36 mmol, 1.00 eq), 8 (1.01 g, 2.73 mmol, 2.00 eq) and BOP (723 mg, 1.63 mmol, 1.20 eq) were dissolved in DCM (5.0 mL). Then, TEA (474 µL, 3.41 mmol, 2.50 eq) was added and the reaction mixture was stirred at 40°C for 16 hours. LCMS analysis after this time showed complete conversion. The reaction mixture was hydrolyzed with water and diluted with DCM. Organic layer was then washed 7 times with saturated aqueous NH₄Cl solution, and 3 times with HCl 1M. The organic layer was then concentrated till dryness and the residue was purified by chromatography over SiO₂ eluting with DCM/MeOH/Et3N (100/0/0.1 to 95/5/0.1). Purest fractions were combined and evaporated to dryness to afford E12 (600 mg, 8.79 mmol, 64% yield) as a clear oil.

LC purity: 90.5 A%, MS (positive ESI, [M+H]+): 683.7, 1H NMR (400 MHz, DMSO, mixture of rotamers) (Figure 12) δ 7.73 - 7.18 (m, 7H), 7.18 - 6.91 (m, 2H), 5.36 - 4.88 (m, 2H), 4.58 - 3.61 (m, 6H), 3.50 - 2.94 (m, 10H), 2.78 (dq, J = 19.8, 7.2 Hz, 2H), 2.19 (ddt, J = 15.7, 11.6, 5.1 Hz, 2H), 1.98 - 1.68 (m, 4H), 1.68 - 1.54 (m, 1H), 1.45 - 1.13 (m, 9H), 1.10 - 0.49 (m, 6H).

### Example 13: Synthesis of a compound of formula F1:

In a 4 mL sealed glass vial, was charged E12 (35.0 mg, 0.051 mmol, 1.00 eq) followed by iPrOH (350 µL). At 25°C, 5.5 M HCl solution in iPrOH (28.0 µL, 0.154 mmol, 3.00 eq), and the reaction was warmed to 50°C for 6h. LCMS analysis after this time showed complete hydrolysis of the Boc group. The reaction mixture was evaporated under reduced pressure and the residue was then dissolved in MeOH (500 µL). Then, Ammonia 7N in solution in MeOH (44.0 µL, 1.99 mmol, 40.00 eq) was added and the reaction mixture was stirred at 50°C for 5 days. The reaction mixture was then evaporated to dryness under reduced pressure and the residue was purified by preparative TLC, eluting with DCM/MeOH 90:10 to afford compound of formula F1 (E7) as a white solid (5.0 mg, 0.010 mmol, 20% yield).

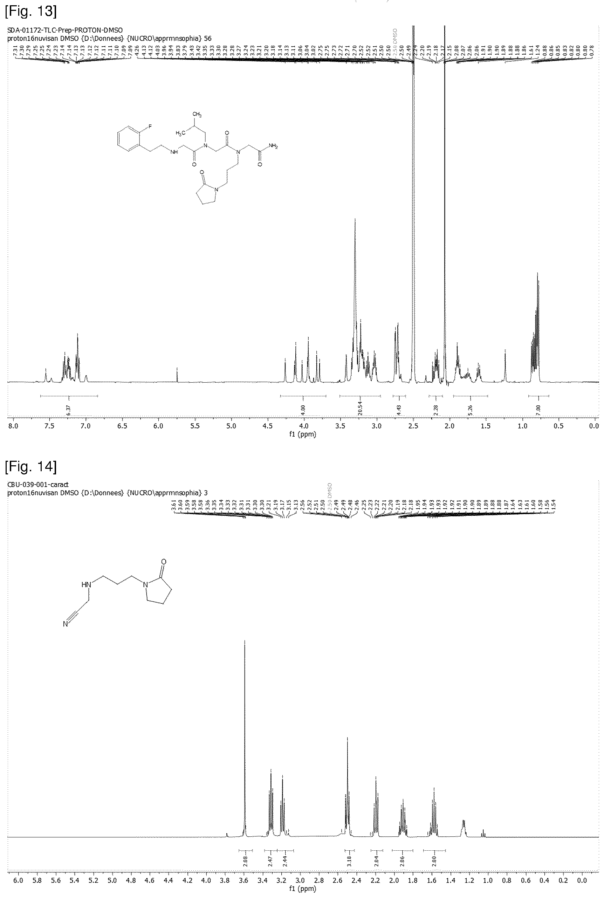

LC purity: 94.0 A%, MS (positive ESI, [M+H]+): 492.4, ¹H NMR (400 MHz, DMSO, mixture of rotamers) (Figure 13) δ 7.70 - 6.88 (m, 7H), 4.37 - 3.68 (m, 4H), 3.46 - 2.99 (m, 10H), 2.79 - 2.63 (m, 4H), 2.41 - 2.11 (m, 2H), 2.05 - 1.43 (m, 5H), 1.10 - 0.55 (m, 6H).

### Example 14: Synthesis of a compound of formula F6:

In a 500mL round bottom flask was introduced 1-(3-aminopropyl)pyrrolidin-2-one (10.0g, 0.070 mol, 1.00 eq) followed by MeCN (360 mL). To this solution was added bromoacetonitrile (8.6 g, 0.07mol, 1.01 eq) (slight exotherm) followed by DIPEA (37.1 mL, 0.21 mol, 3.03 eq). The solution was stirred for 18h.

LCMS analysis after this time showed full conversion with formation of both mono and bis-alkylated product in a 93/17 ratio. The solution was quenched by addition of water (50 mL) and DCM (150 mL). Layers were separated and the aqueous phase was extracted DCM (3x50 mL DCM). Combined organic phases were washed with a saturated aqueous NaCl solution (2x30 mL), dried over Na₂SO₄ and concentrated to dryness under reduced pressure to afford 8.5g of a yellowish oil. This residue was purified by chromatography over SiO₂ eluting with DCM/EtOH/Et3N (97/3/0.1). Purest fractions were combined and evaporated to dryness to afford E14 (6.2g, 48% yield) as a colorless oil.

LC purity: 97.0 A%, MS (positive ESI, [M+H]+): 182.2,¹H NMR (400 MHz, DMSO) (Figure 14) δ 3.60 (s, 2H), 3.38 - 3.29 (m, 2H), 3.20 (t, J = 7.1 Hz, 2H), 2.54 - 2.49 (m, 3H), 2.21 (dd, J = 8.7, 7.5 Hz, 2H), 1.98 - 1.86 (m, 2H), 1.59 (p, J = 7.0 Hz, 2H).

### Example 15: Synthesis of a compound of formula F2:

In a 100mL round bottom flask was introduced E9 (0.750g, 0.002mol, 1.00 eq) followed by DCM (360mL). To this solution was added triethylamine (0.300mL, 0.002mol, 1.00 eq) followed by E14 (0.603g, 0.003mol, 1.50 eq). Finally, BOP (1.5g, 0.003 mol, 1.50 eq) was added to the solution followed by triethylamine (0.300 mL, 0.002 mol, 1.00 eq). The solution was stirred at 40°C for 2h.

LCMS analysis after this time showed full conversion with. The solution was quenched by addition of water (10mL) and DCM (10mL). Layers were separated and the organic phase was washed with water (4x20mL) then with 1N aqueous HCl (2x20mL) and finally with a saturated aqueous NaCl solution (1x20mL). The organic phase was dried over Na₂SO₄ and concentrated to dryness under reduced pressure to afford a yellowish oil. This residue was purified by thin layer chromatography over SiO₂ eluting with DCM/EtOH/Et3N (93/7/0.1). Purest fractions were combined and evaporated to dryness to afford E15 (530mg, 48% yield) as a colorless oil.

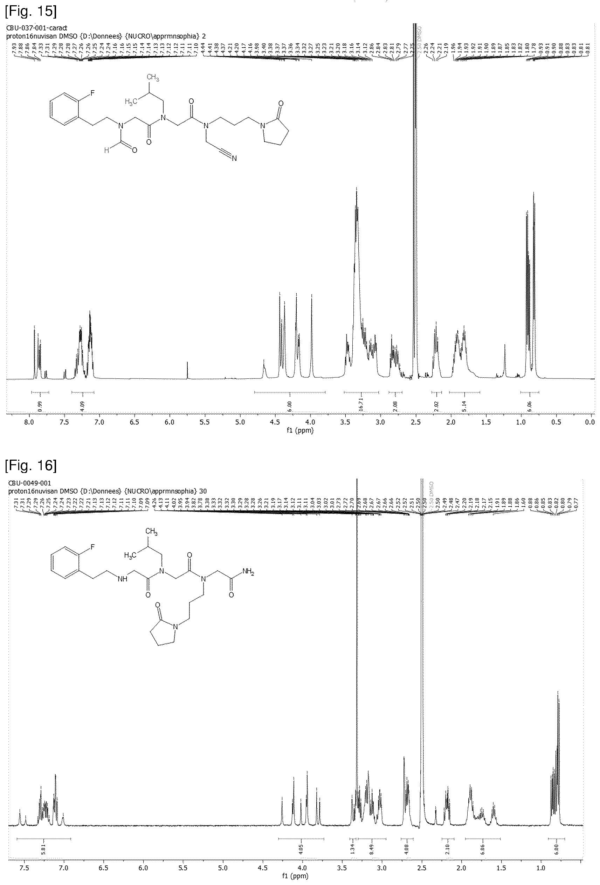

LC purity: 94.2 A%, MS (positive ESI, [M+H]+): 502.4, ¹H NMR (400 MHz, DMSO, mixture of rotamers) (Figure 15) δ 8.35 - 7.73 (m, 1H), 7.45 - 6.68 (m, 4H), 5.04 - 3.84 (m, 6H), 3.60 - 2.98 (m, 10H), 2.89 - 2.72 (m, 2H), 2.24 (q, J = 8.8 Hz, 2H), 2.01 - 1.77 (m, 5H), 0.99 - 0.73 (m, 6H).

### Example 16: Synthesis of a compound of formula F1:

In a 25mL round bottom flask was introduced E15 (0.50 g, 0.001 mol, 1.00 eq) followed by MeOH (3.0 mL) and conc. H2SO4 (0.22 mL, 0.004 mol, 5.00 eq). The solution was stirred at 50°C for 16h.

LCMS analysis after this time showed complete conversion into the corresponding methyl ester. The reaction was diluted with DCM (10 mL) and a 2.5M K₃PO₄ aqueous solution (2.6 mL) was added. Layers were separated and the organic phase was washed with water (2x10 mL) and then with 1N HCl (2x10 mL). The organic phase was then dried over Na₂SO₄ and concentrated to dryness under reduced pressure to afford yellow oil.

This residue was dissolved in MeOH (0.5 mL) and a 7M NH3 solution in MeOH was added (1.5 mL). The solution was stirred at 50°C for 24h in a sealed tube.

LCMS analysis after this time showed complete conversion into compound of formula F1 (E7). The solution was evaporated to dryness under reduced pressure and the residue was diluted in EtOAc (5 mL) and warmed to 50°C. The solution was stirred at 50°C for 1h and allowed to cool to 23°C for 24h while the product crystallized. The solid was collected by filtration, washed with DIE (5 mL) and dried under vacuum at 45°C for 24h to afford compound of formula F1 (E7) (100mg, 45% yield).

LC purity: 98.9 A%, MS (positive ESI, [M+H]+): 492.4, ¹H NMR (400 MHz, DMSO, mixture of rotamers) (figure 16) δ 7.70 - 6.88 (m, 7H), 4.37 - 3.68 (m, 4H), 3.46 - 2.99 (m, 10H), 2.79 - 2.63 (m, 4H), 2.41 - 2.11 (m, 2H), 2.05 - 1.43 (m, 5H), 1.10 - 0.55 (m, 6H).

### Example 17: Synthesis of a compound of formula F2:

In a 25mL round bottom flask were introduced E4 (1.00 g, 0.002 mol, 1.00 eq) and E14 (0.83 g, 0.005mol, 2.00 eq) followed by DCM (8.4 mL). To this solution was added triethylamine (0.79 mL, 0.006 mol, 2.50 eq) and BOP (1.52 g, 0.003 mol, 1.50 eq) and the reaction was stirred at 50°C for 16h.

LCMS analysis after this time showed full conversion. The reaction was quenched by addition of water (10.0mL) and DCM (10.0mL). Layers were separated and the organic phase was washed once with water (10.0mL), then with a NH₄Cl saturated aqueous solution (3x10mL). The organic phase was washed with water (10.0mL), dried on Na₂SO₄ and evaporated to dryness under reduced pressure to afford a yellowish oil which was purified by chromatography on SiO₂ using DCM/EtOH (96/4) as eluant. Purest fractions were combined and evaporated to dryness to afford E17 (585mg, 42% yield) as a colorless oil.

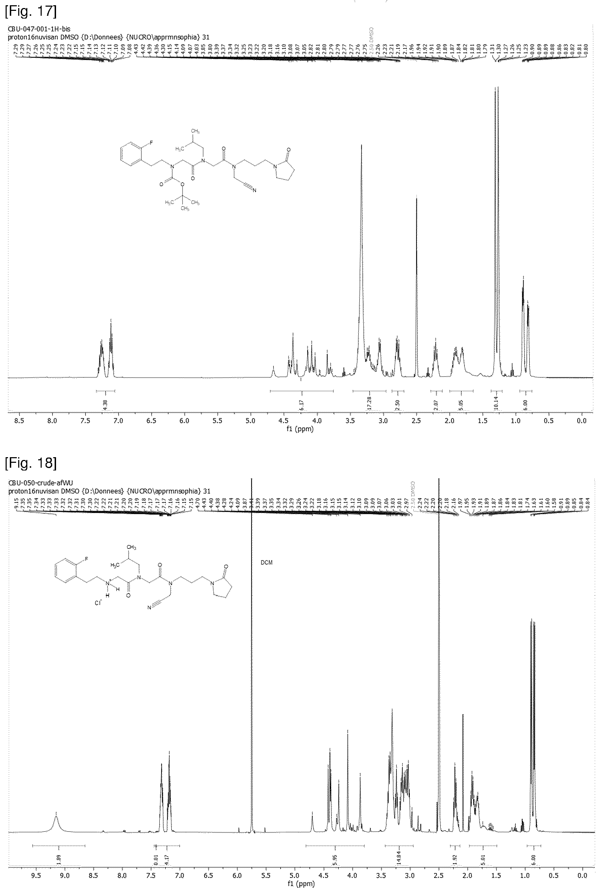

LC purity: 93.7 A%, MS (positive ESI, [M+H]+): 574.4, ¹H NMR (400 MHz, DMSO, mixture of rotamers) (figure 17) δ 7.47 - 6.98 (m, 4H), 4.73 - 3.72 (m, 6H), 3.53 - 2.94 (m, 10H), 2.91 - 2.67 (m, 2H), 2.29 - 2.18 (m, 2H), 2.06 - 1.69 (m, 5H), 1.45 - 1.16 (m, 9H), 0.94 - 0.75 (m, 6H)

### Example 18: Synthesis of a compound of formula F2:

In a 20mL round bottom flask was introduced E17 (0.50 g, 0.001 mol, 1.00 eq) followed by iPrOH (1.0mL) and 5.5N HCl in iPrOH (0.50 mL, 0.003 mol, 4.50 eq). The reaction mixture was stirred at 23°C for 20h.

LCMS analysis after this time showed full conversion. Water (5.0mL) and DCM(5.0mL) were added. Layers were separated, and the aqueous layer was extracted with DCM (5x30mL), Organic layers were combined then dried over Na₂SO₄ and evaporated to dryness to afford E18.HCl (0.09g, 29% yield) as a colorless oil.

LC purity: 93.2 A%, MS (positive ESI, [M+H]+): 474.4, ¹H NMR (400 MHz, DMSO, mixture of rotamers) (figure 18) δ 9.16 (br, 2H), 7.42 - 7.28 (m, 2H), 7.25 - 7.05 (m, 2H), 4.87 - 3.75 (m, 6H), 3.56 - 2.84 (m, 12H), 2.32 - 2.13 (m, 2H), 1.99 - 1.54 t(m, 5H), 0.88 (dd, J = 21.1, 6.6 Hz, 6H).

### Example 19: Synthesis of a compound of formula F1:

In a 8mL glass vial was charged E18 (100 mg, 0.211 mmol, 1.00 eq) followed by MeOH (1.0mL). Then a 7M NH₃ solution in MeOH was added (0.50 mL) and the reaction mixture was stirred at 40°C for 72h. After this time, the reaction mixture was cooled to 23°C and evaporated to dryness under reduced pressure. The residue was purified by chromatography on SiO₂ using DCM/MeOH (98/2) as eluant. Purest fractions were combined and evaporated to dryness to afford compound of formula F1 (E7) (6 mg, 6% yield) as a white solid.

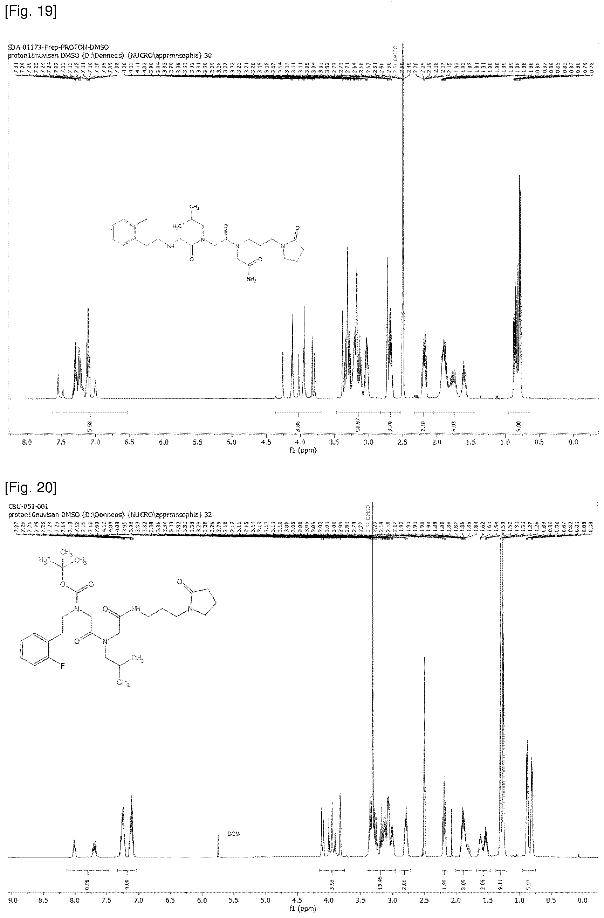

LC purity: 98.1 A%, MS (positive ESI, [M+H]+): 492.4, ¹H NMR (400 MHz, DMSO, mixture of rotamers) (figure 19) δ 7.70 - 6.88 (m, 7H), 4.37 - 3.68 (m, 4H), 3.46 - 2.99 (m, 10H), 2.79 - 2.63 (m, 4H), 2.41 - 2.11 (m, 2H), 2.05 - 1.43 (m, 5H), 1.10 - 0.55 (m, 6H).

### Example 20: Synthesis of a compound of formula F24:

In a 25mL round-bottom flask was introduced 5 (0.50 g, 0.001 mol, 1.00 eq) followed by DCM (2.3mL). Then 1-(3-aminopropyl)pyrrolidine-2-one (0.33 g, 0.002 mol, 2.00 eq) was added, followed by BOP (0.72 g, 0.002 mol, 1.40 eq) and Et3N (0.32 mL, 0.002 mol, 2.00 eq). The reaction mixture was stirred at 40°C for 16h.

LCMS analysis after this time showed complete conversion. The reaction was quenched by addition of DCM (10 mL) and water (10 mL). Layers were separated and the aqueous phase was extracted DCM (2x10mL). Combined organic phases were washed with a saturated aqueous NaCl solution (2x15mL), dried over Na₂SO₄ and concentrated to dryness under reduced pressure. The residue was purified by chromatography over SiO₂ eluting with DCM/EtOH 96:4. Purest fractions were combined and evaporated to dryness to afford E20 (0.34g, 51% yield) as a colorless oil.

LC purity: 95.9 A%, MS (positive ESI, [M+H]+): 535.4, ¹H NMR (400 MHz, DMSO, mixture of rotamers) (figure 20) δ 8.05 - 7.49 (m, 1H), 7.24 - 7.12 (m, 2H), 7.11 - 6.99 (m, 2H), 4.22 - 3.56 (m, 4H), 3.33 - 2.89 (m, 10H), 2.78 - 2.67 (m, 2H), 2.16 - 2.07 (m, 2H), 1.89 - 1.69 (m, 3H), 1.61 - 1.40 (m, 2H), 1.26 - 1.15 (m, 9H), 1.11 - 0.59 (m, 6H).

### Example 21: Synthesis of a compound of formula F1:

In a 8mL vial was introduced E20 (100 mg, 0.131mmol, 1.00 eq) followed by THF (330 µL). LiHMDS (0.131mL of a 1M solution in THF, 0.131mmol, 1.0 eq) was added dropwise and the reaction mixture was let stirred at 23°C for 15min before methyl-bromoacetate (0.05 mL, 0.524 mmol, 4.0 eq) addition. The reaction mixture was stirred at 23°C for 18h.

The reaction was quenched by addition of DCM (5 mL) and water (5 mL). Layers were separated and the organic layer was washed once with a saturated NH₄Cl aqueous solution then with brine, dried over Na₂SO₄ and evaporated until dryness under reduce pressure to afford a colorless oil (240 mg). This crude oil was placed in a 4mL round bottom flask followed by MeOH (600 µL) and H₂SO₄ (70 µL). The reaction mixture was stirred at 23°C for 18h. LCMS analysis after this time showed complete Boc deprotection. The reaction was diluted with DCM (3 mL) and quenched with a 2.5 N K₃PO₄ aqueous solution (1mL). Phases were separated and the organic phase was washed with brine (5mL), dried over Na₂SO₄ and evaporated until dryness under reduced pressure. The residue was then dissolved in MeOH (500 µL). Ammonia 7N in solution in MeOH (100 µL) was added and the reaction mixture was stirred at 50°C for 5 days. The reaction mixture was then evaporated to dryness under reduced pressure and the residue was purified by preparative TLC, eluting with DCM/MeOH 90:10 to afford compound of formula F1 (E7) as a white solid (5.0 mg, 0.010 mmol, 20% yield over 3 steps).

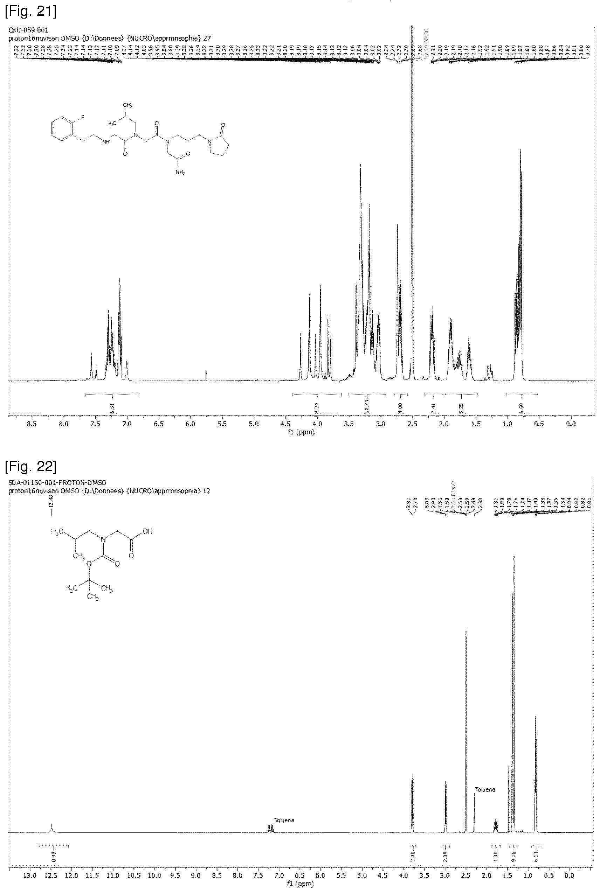

LC purity: 98.1 A%, MS (positive ESI, [M+H]+): 492.4, ¹H NMR (400 MHz, DMSO, mixture of rotamers) (figure 21) δ 7.70 - 6.88 (m, 7H), 4.37 - 3.68 (m, 4H), 3.46 - 2.99 (m, 10H), 2.79 - 2.63 (m, 4H), 2.41 - 2.11 (m, 2H), 2.05 - 1.43 (m, 5H), 1.10 - 0.55 (m, 6H).

### Example 22: Synthesis of a compound of formula F20:

In a 250 mL round bottom flask was added E3 (7.00 g, 0.042 mol, 1.00 eq) followed by EtOH (50 mL). At 25°C, TEA was added (7.0 mL, 0.050 mol, 1.20 eq) followed by a solution of Boc₂O (9.1 g, 0.042 mol, 1.00 eq) solubilized in EtOH (20 mL). The reaction solution was then stirred at 25°C for 16 hours. The reaction mixture was then concentrated till dryness under reduced pressure and the residue diluted with DCM and water. Phases were separated and the organic phase was washed with 0.05M aqueous HCl and with water. The organic layer was dried over Na₂SO₄, filtered, and concentrated till dryness under reduced pressure. Residual EtOH was swapped several times with Toluene to afford E22 (8.15 g, 0.035 mol, 84% yield) as a colorless oil containing ~6% w/w of toluene.

¹H NMR (400 MHz, DMSO) (figure 22) δ 12.49 (s, 1H), 3.80 (d, J = 10.1 Hz, 2H), 3.00 (d, J = 7.2 Hz, 2H), 1.79 (dq, J = 13.9, 6.9 Hz, 1H), 1.37 (d, J = 16.8 Hz, 9H), 0.83 (dd, J = 6.6, 4.7 Hz, 6H).

### Example 23: Synthesis of a compound of formula F28:

In a 250 mL round bottom single neck flask was charged E22 (4.00 g, 17.3 mmol, 1.00 eq), E8.HBr (6.42 g, 17.3 mmol, 1.00 eq) followed by DCM (80 mL). At 25°C, EtsN (7.2 mL, 52 mmol, 3.00 eq) was added, followed by BOP (11.47 g, 26 mmol, 1.50 eq). The reaction mixture was then stirred at 40°C for 2 hours and then at 25°C for 16 hours. LCMS analysis showed almost complete conversion after this time.

The reaction mixture was then hydrolyzed with a 1M aqueous NaHCOs solution. Phases were separated and the organic layer was washed with water and concentrated till dryness to afford an orange oil. The residue was solubilized in 2-MeTHF / Heptane 85:15. The organic layer was warmed to 55°C and washed with water twice. The organic layer was then concentrated till dryness and the residue was purified by column chromatography on SiO₂ eluting with DCM/EtOAc first and then with DCM/MeOH. Purest fractions were combined and concentrated to afford E23 (5.50 g, 63% yield) as a yellow liquid.

LC purity: 94.4 A%, MS (positive ESI, [M+H]+): 504.5, ¹H NMR (400 MHz, DMSO, mixture of rotamers) (figure F23) δ 7.52 - 7.12 (m, 5H), 5.38 - 4.96 (m, 2H), 4.60 - 3.73 (m, 4H), 3.57 - 3.07 (m, 6H), 3.05 - 2.75 (m, 2H), 2.31 - 2.11 (m, 2H), 1.97 - 1.53 (m, 5H), 1.45 - 1.25 (m, 9H), 0.90 - 0.71 (m, 6H).

### Example 24: Synthesis of a compound of formula F3:

In a 500 mL flask was introduced 2-(2-fluorophenyl)ethan-1-amine (5.6 mL, 0.043 mol, 1.00 eq) followed with H2O (36.0 mL), IPA (9.0 mL) and formic acid (2.58 mL, 0.060 mol, 1.40 eq). Formic acid addition led to an exotherm and internal temperature reached 33°C. After cooling at 25°C, was added dropwise in 15min a glyoxylic acid (11.9 g, 0.130 mol, 3.00 eq) solution in H2O (18.0 mL) (no exotherm observed) and reaction was stirred at 50°C for 18 hours. The suspension was allowed to slowly come back at 23°C and was stirred for 1 h at 23°C. The solid was collected by filtration, washed with water and dried under vacuum at 45°C overnight to afford E24 (7.98 g, 0.036 mol, 82% yield) as a white solid.

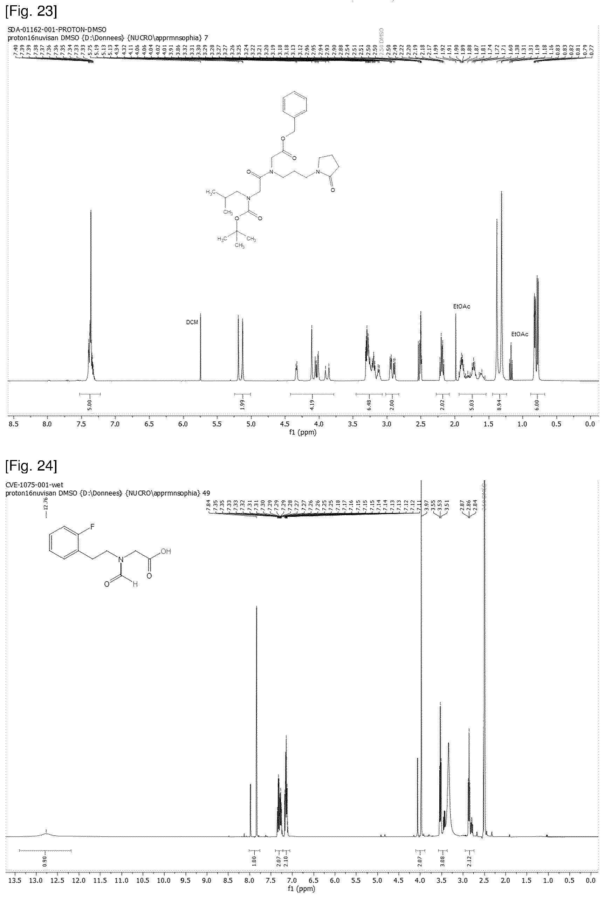

LC purity: 97.3 A%, MS (positive ESI, [M+H]+): 226.2, ¹H NMR (400 MHz, DMSO) (figure 24) δ 12.77 (s, 1H), 7.85 (s, 1H), 7.42 - 7.20 (m, 3H), 7.23 - 6.96 (m, 3H), 3.98 (s, 2H), 3.54 (t, J = 7.0 Hz, 2H), 2.86 (t, J = 7.0 Hz, 2H).

### Example 25: Synthesis of a compound of formula F2:

In a 25 mL round bottom flask was charged E23 (310 mg, 0.616 mmol, 1.000 eq) followed by iPrOH (1.5 mL). At 25°C, HCl 5.5 N solution in iPrOH (448 µL, 2.46 mmol, 4.000 eq) was added and the reaction solution was stirred at 50°C for 1 hour and then at 23°C for 16 hours. The reaction mixture was then concentrated to dryness under reduced pressure. The residue was suspended in DCM (5.0 mL) and E24 (57.6 mg, 0.256 mmol, 1.000 eq) was added followed by BOP (169.6 mg, 0.384 mmol, 1.500 eq). The reaction mixture was heated at 40°C, and EtsN (142 µL, 1.023 mmol, 4.000 eq) was added. The reaction mixture was stirred at 40°C for 1h. LCMS analysis showed complete conversion after this time. The reaction mixture was hydrolyzed with water, phases were separated, and the organic layer was washed with water, dried over Na₂SO₄ filtered and concentrated till dryness under reduced pressure. The residue was purified by chromatography over SiO₂ eluting with DCM / EtOH 95:5 to afford E10 (76mg, 0.125 mmol, 22% yield) as a white solid.

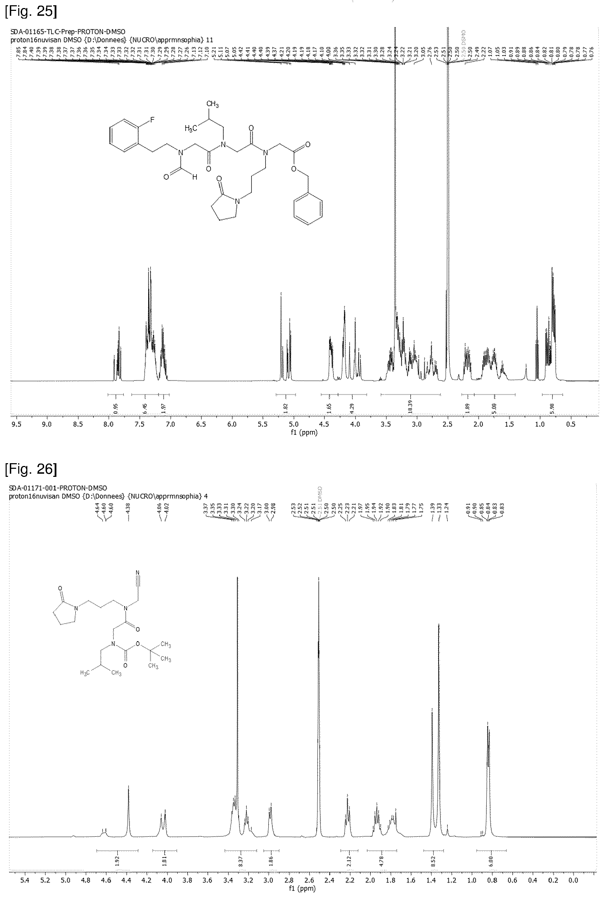

LC purity: 92.3 A%, MS (positive ESI, [M+H]+): 611.5, ¹H NMR (400 MHz, DMSO, mixture of rotamers) (figure 25) δ 8.05 - 7.72 (m, 1H), 7.55 - 7.22 (m, 7H), 7.16 - 6.98 (m, 2H), 5.38 - 4.92 (m, 2H), 4.56 - 3.88 (m, 6H), 3.60 - 2.62 (m, 12H), 2.26 - 2.10 (m, 2H), 1.97 - 1.67 (m, 4H), 1.68 - 1.51 (m, 1H), 0.93 - 0.74 (m, 6H).

### Example 26: Synthesis of a compound of formula F28:

In a 25 mL round bottom flask, was loaded E14 (380 mg, 2.09 mmol, 1.00 eq) and E22 (485 mg, 2.09 mmol, 1.00 eq) followed by DCM (3.8 mL) and BOP (1.39 g, 3.14 mmol, 1.50 eq). The reaction mixture was warmed to 40°C and Et3N (0.58 mL, 4.193 mmol, 2.00 eq) was added. The reaction mixture was then stirred at 40°C for 2 hours.

LCMS analysis showed complete conversion after this time. The reaction mixture was hydrolyzed with a 1N aqueous NaHCOs solution water and phases were separated. The organic layer was washed a 1M HCl solution, then with water and evaporated to dryness under reduced pressure. The residue was purified by chromatography over SiO₂ eluting with DCM/EtOAc 50:50 and then DCM/EtOH 95:5. Purest fractions were combined and evaporated to dryness under reduced pressure to afford E26 (330 mg, 0.836 mmol, 40% yield) as a yellow oil.

MS (positive ESI, [M+H]+): 395.4, ¹H NMR (400 MHz, DMSO, mixture of rotamers) (figure 26) δ 4.80 - 4.28 (m, 2H), 4.04 (d, J = 15.0 Hz, 2H), 3.51 - 3.10 (m, 6H), 3.06 - 2.81 (m, 2H), 2.23 (t, J = 8.0 Hz, 2H), 2.07 - 1.61 (m, 5H), 1.36 (d, J = 25.7 Hz, 9H), 0.85 (d, J = 2.9 Hz, 6H).

### Example 27: Synthesis of a compound of formula F27:

In a 100 mL round bottom flask, was loaded E14 (3.15 g, 17.38 mmol, 1.00 eq) followed by DCM (31.5 mL). At 25°C, EtsN (3.1 mL, 22.59 mmol, 1.30 eq) was added followed by chloroacetyl chloride (1.4 mL, 17.38 mmol, 1.000 eq). The reaction mixture was then stirred at 25°C for 1 hour.

LCMS analysis showed complete conversion after this time. The reaction mixture was hydrolyzed with water and phases were separated. The organic layer was washed with water and then concentrated in a rotary evaporator till dryness to afford E27 (3.53 g, 13.70 mmol, 79% yield) as an orange oil, which crystallizes overtime.

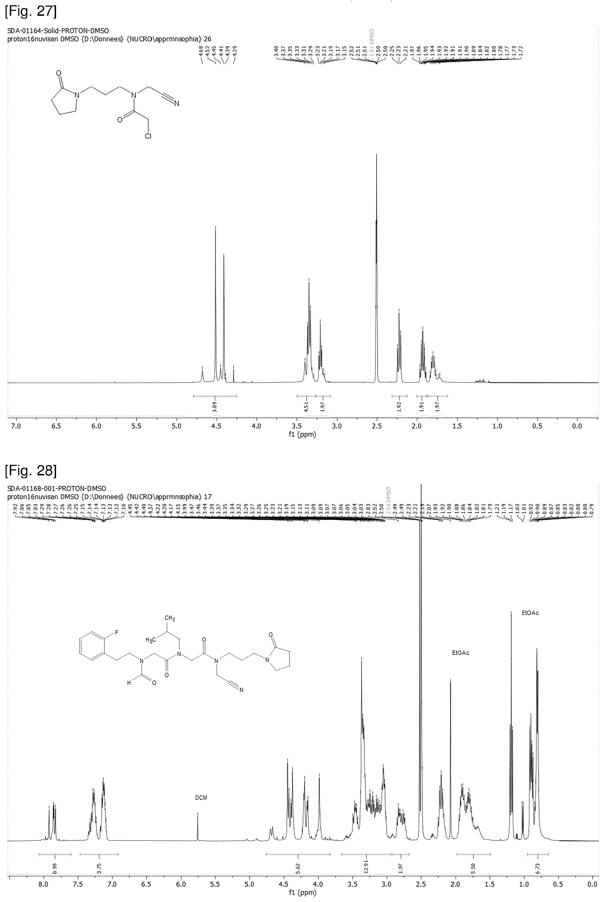

LC purity: 97.8 A%, MS (positive ESI, [M+H]+): 258.3, ¹H NMR (400 MHz, DMSO, mixture of rotamers) (figure 27) δ 4.98 - 4.08 (m, 4H), 3.58 - 3.27 (m, 4H), 3.21 (t, J = 6.8 Hz, 2H), 2.23 (t, J = 8.0 Hz, 2H), 1.93 (p, J = 7.4 Hz, 2H), 1.88 - 1.59 (m, 2H).

### Example 28: Synthesis of a compound of formula F2:

In a 8 mL glass vial was charged E26 (550.0 mg; 1.39 mmol; 1.00 eq) followed iPrOH (5.0 mL). At 25°C, 5-6N HCl in iPrOH (1.0 mL; 5.6 mmol; 4.00 eq) was added and reaction solution was heated up to 40°C for 16 hours.

LCMS analysis showed complete conversion after this time. The reaction mixture was evaporated to dryness under reduced pressure to afford a pale yellow oil (448 mg). The residue was solubilized in DCM (5.0 mL) and E24(343 mg, 1.522 mmol, 1.00 eq) and BOP (1.00 g, 2.283 mmol, 1.50 eq) were added. This solution was then heated near 40°C, and EtsN (423 µL; 3.043 mmol; 2.000 eq) was added. The reaction mixture was stirred at 40°C for 1h. LCMS analysis showed complete conversion after this time. The reaction mixture was concentrated till dryness, diluted with 2-MeTHF, and washed with a saturated aqueous NaHCOs solution. The organic layer was concentrated till dryness to afford an orange oily residue, which was purified by chromatography over SiO₂ eluting with DCM / EtOAc 1/1 to afford E15 as a colorless oil (120 mg,0.24 mmol, 17% yield over 2 steps).

LC purity: 96.3 A%, MS (positive ESI, [M+H]+): 502.4, ¹H NMR (400 MHz, DMSO, mixture of rotamers) (figure 28) δ 8.35 - 7.73 (m, 1H), 7.45 - 6.68 (m, 4H), 5.04 - 3.84 (m, 6H), 3.60 - 2.98 (m, 10H), 2.89 - 2.72 (m, 2H), 2.24 (q, J = 8.8 Hz, 2H), 2.01 - 1.77 (m, 5H), 0.99 - 0.73 (m, 6H).

### Example 29: Synthesis of a compound of formula F2:

In a 100 mL round bottom flask was charged E27 (3.20 g; 12 mmol; 1.00 eq) followed by MeCN (25.0 mL). To this solution was then added EtsN (2.1 mL; 15 mmol; 1.20 eq) followed few minutes later by isobutylamine (1.4 mL; 14 mmol; 1.10 eq). The reaction mixture was stirred at 23°C for 18 hours.

LCMS analysis showed complete conversion after this time. The reaction mixture was concentrated under reduced pressure to a low volume and diluted with 2-MeTHF and water. Phases were separated and the organic layer was washed with water. Aqueous layers were combined and extracted 3 times with DCM/EtOH 80:20. Organic layers (2-MeTHF and DCM/EtOH) were combined, dried over Na₂SO₄ and evaporated to dryness under reduced pressure to afford a brown oil (2.2 g).

400mg of this crude compound were solubilized in DCM (5.0 mL). At 25°C, EtsN (420 µL; 3.020 mmol) was added, followed by Chloroacetyl chloride (110 µL; 1.379 mmol). This solution was stirred at 25°C for 30 minutes. LCMS analysis showed complete conversion after this time. The reaction mixture was hydrolyzed with water and phases were separated. The organic phase was dried over Na₂SO₄ and evaporated to dryness under reduced pressure to afford a brown oil (550 mg). This residue was solubilized in MeCN (2.5 mL). At 25°C, EtsN (155 µL; 1.112 mmol) was added, followed by a solution of 2-(2-fluorophenyl)ethan-1-amine (97 µL ; 0.741 mmol) in MeCN (1.3 mL). The reaction mixture was then stirred at 25°C for 16 hours. LCMS analysis showed complete conversion after this time. The reaction mixture was then evaporated under reduced pressure till dryness and the residue was diluted with 2-MeTHF and washed with an aqueous NaHCOs solution. The organic phase was dried over Na₂SO₄ and evaporated to dryness under reduced pressure to afford an oily orange residue which was purified by chromatography over SiO₂ eluting with DCM/EtOH 9:1 to afford E18 as a colorless oil (100 mg; 28% yield over 3 steps).

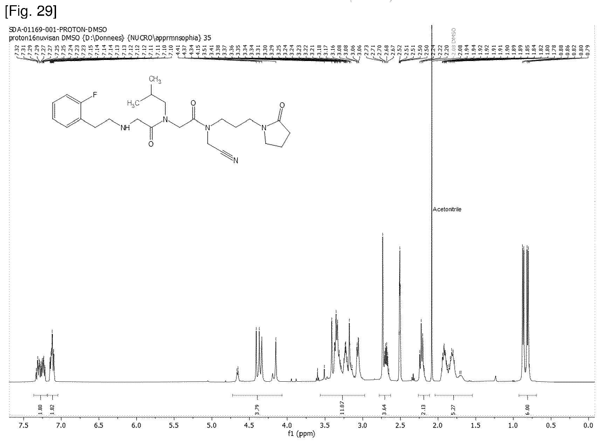

LC purity: 95.1 A%, MS (positive ESI, [M+H]+): 474.4, ¹H NMR (400 MHz, DMSO, mixture of rotamers) (figure 29) δ 7.38 - 7.18 (m, 2H), 7.18 - 7.07 (m, 2H), 4.92 - 4.02 (m, 4H), 3.56 - 2.96 (m, 11H), 2.79 - 2.59 (m, 3H), 2.22 (t, J = 8.0 Hz, 2H), 2.05 - 1.53 (m, 5H), 0.84 (dd, J = 23.9, 6.6 Hz, 6H).

## Claims

1. A process for synthesizing a compound of formula F1: 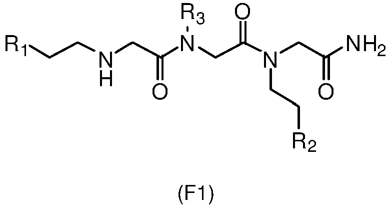 wherein:
R₁ is phenyl substituted with halogen or trifluoromethyl, and further optionally substituted with one or
two substituents selected from the group consisting of halogen, (C₁-C₆)alkyl, (C₁-C₆)alkoxy, and
halo(C₁-C₆)alkyl; or alternatively R₁ is pyrrolidin-1-yl;
R₂ is 2-oxo-pyrrolidin-1-ylmethyl or sulfamoylphenyl; and
R₃ is chosen from propyl, 1-methylethyl, butyl, 2-methylpropyl, pentyl, 1 -methyl-butyl, 2- methylbutyl, hexyl, 4-methylpentyl, 3-methylpentyl, 2-methylpentyl, and 1- methylpentyl;
comprising the steps of:
(S1) synthesizing a compound of formula F2: 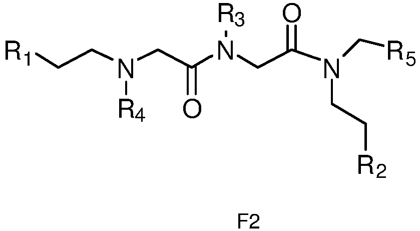
wherein R₄ is hydrogen atom, or a protecting group selected in the group comprising, Boc, Fmoc or formyl,
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
(S2) if R₄ is a protecting group selected in the group comprising, Boc, Fmoc or formyl, removing said protecting group,
(S3) Amidifying R₅.

2. Process according to claim 1, wherein step (S1) comprises the following successive sub steps:
(S1-1a) reacting a compound of formula F3 with a compound of formula F4: 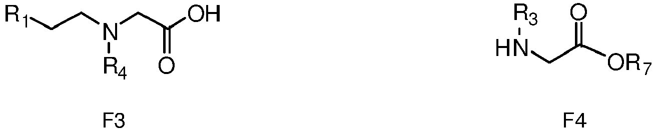
wherein R₇ being -CH₃, -CH₂CH₃; ⁱPr; -^{t}Bu, -H, or -Benzyl,
resulting in a compound of formula F5 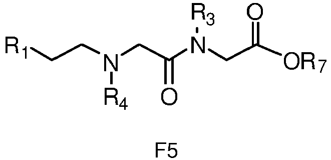
(S1-1b) reacting F5 with a compound or formula F6

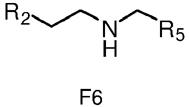

3. Process according to claim 2, wherein step (S1-1a) comprises the following successive sub steps:
(S1-1aa) reacting a compound of formula F3 with a compound of formula F7: 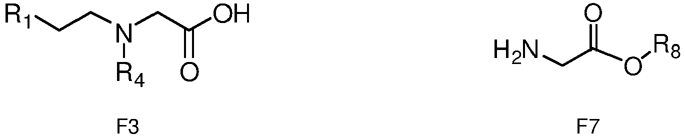
wherein R₈ being -CH₃, -CH₂CH₃; ⁱPr; -^{t}Bu, -H, or -Benzyl,
resulting in a compound of formula F8 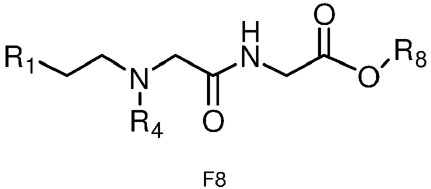 (S1-1ab) reacting F8 with a compound or formula F9 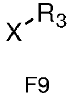 wherein X is a halogen atom,
resulting in a compound of formula F10 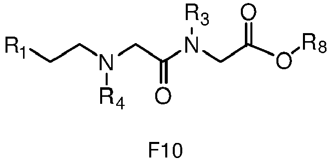
(S1-1ac) if R₈ is different from H, and R₇ is H, hydrolyzing F10 resulting in a compound of formula F5.

4. Process according to any one of claims 2 and 3, wherein compound of formula F6 is synthesized by reacting a compound of formula F11 with a compound of formula F12 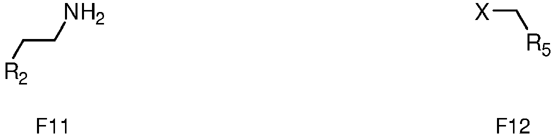 wherein X is a halogen atom.

5. Process according to any one of claims 2 to 4, wherein compound of formula F3 is synthesized by reacting a compound of formula F13 with a compound of formula F14 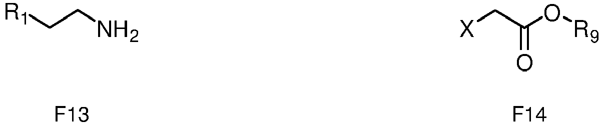
wherein X is a halogen atom, and
wherein R₉ being -CH₃, -CH₂CH₃; ⁱPr; -^{t}Bu, -H, or -Benzyl,
resulting in a compound of formula F15, 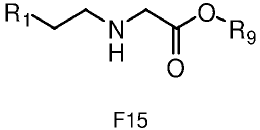
protecting the amine function of said compound of formula F15 with a protecting group selected in the group comprising, Boc, Fmoc and formyl, resulting in a compound of formula F16 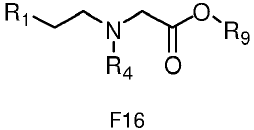 hydrolyzing F16.

6. Process according to claim 1, wherein step (S1) comprises the following successive sub steps:
(S1-2a) reacting a compound of formula F17 with a compound of formula F3:

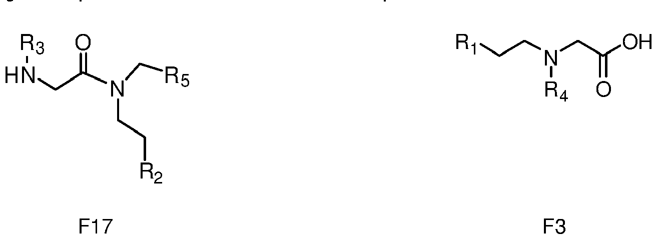

7. Process according to claim 1, wherein step (S1) comprises the following successive sub steps:
(S1-3a) reacting a compound of formula F17 with a compound of formula F18: 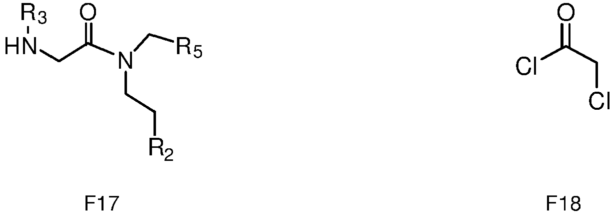
resulting in a compound of formula F19 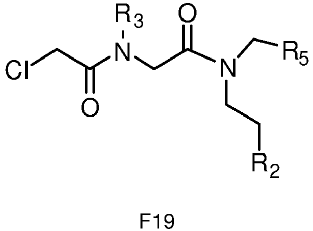
reacting F19 with a compound of formula F13:

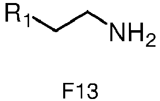

8. Process according to anyone of claims 6 and 7, wherein compound of formula F17 is synthesized by reacting a compound of formula F6 with a compound of formula F20 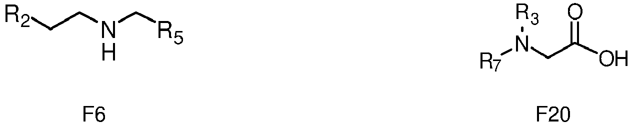
wherein R₇ is a protecting group selected from BOC, FMOC, formyl or benzyl, and
removing said protecting group.

9. Process according to anyone of claims 6 and 7, wherein compound of formula F17 is synthesized by reacting a compound of formula F6 with a compound of formula F18 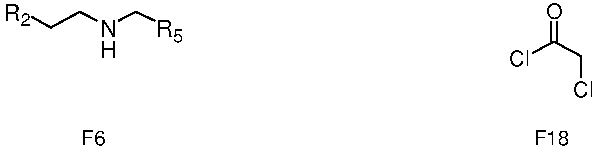
resulting in a compound of formula F21 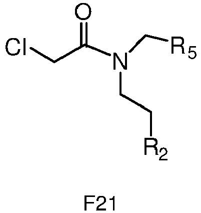
reacting F21 with a compound of formula F22

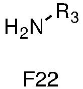

10. A Compound of formula F23: 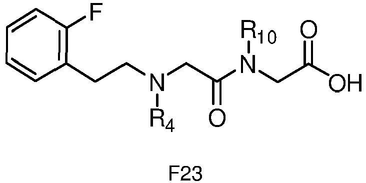
wherein R₁₀ is -H or isobutyl,
and salts thereof.

11. A Compound of formula F24: 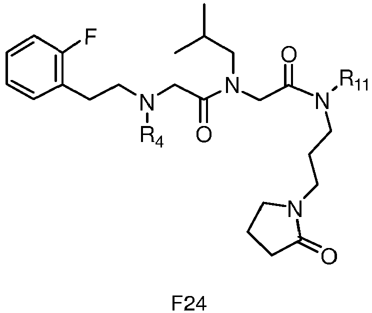
wherein R₁₁ is -H or -CH₂R₅,
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof.

12. A Compound of formula F25: 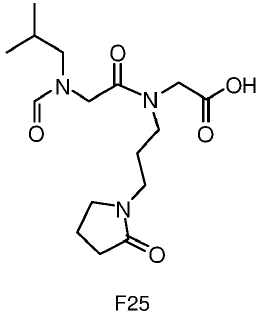 and salts thereof.

13. A Compound of formula F26: 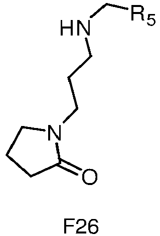
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof.

14. A compound of formula F27: 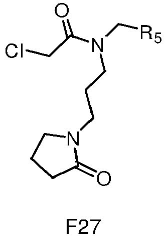
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof.

15. A compound of formula F28: 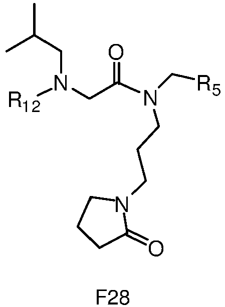
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl, and
wherein R12 is hydrogen atom, BOC, FMOC, formyl, Bn or COCH₂Cl,
and salts thereof.

16. A Compound of formula F29: 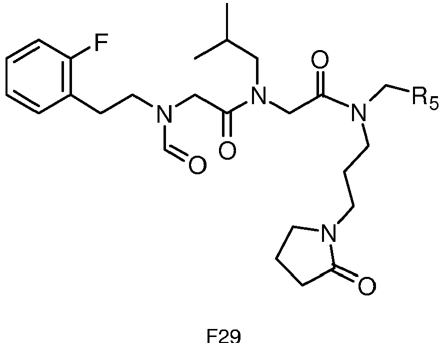
wherein R₅ is -CN, -C(O)NH₂, -C(O)NHCH₂Ph or -C(O)OR₆, R₆ being -CH₃, -CH₂CH₃; ⁱPr; -^{t}Bu, -H, or-Benzy,
and salts thereof.

17. A Compound of formula F30: 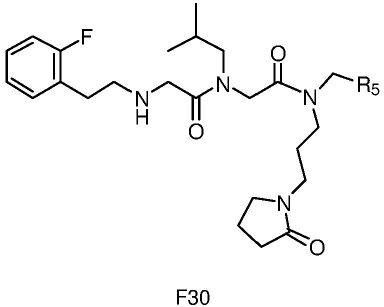
wherein R₅ is -CN, -C(O)NH₂, -C(O)NHCH₂Ph or -C(O)OR₆, R₆ being -CH₃, -CH₂CH₃; ⁱPr; -^{t}Bu, -H, or -Benzyl,
and salts thereof.

18. A Pharmaceutical composition comprising the compound of formula F1 obtained by the process according to anyone of claims 1 to 9, and optionally one or more pharmaceutically acceptable excipient(s).
